# EUROPEAN PATENT APPLICATION

(11) **EP 4 585 181 A1**
(43) Date of publication of application: **16.07.2025**
(21) Application number: 24167051.2
(22) Date of filing: 27.03.2024
(51) Int. Cl.: A61B 34/30, A61B 90/90, A61B 18/14, A61B 17/00, A61B 18/00, A61B 18/22

(54) **IMPROVED SURGERY SYSTEM**

(30) Priority: 15.01.2024 EP 24151941
(71) Applicant: Symphera GmbH, 81675 München (DE)
(72) Inventor: Preißler, Philipp, 81371 München (DE); Wegele, Thomas, 85748 Garching (DE); Heid, Conrad, 4053 Basel (CH)
(74) Representative: Stellbrink & Partner Patentanwälte mbB

(57) **Abstract**

Disclosed is a system comprising a surgery device, a tool set comprising a plurality of tools and a power supply. The power supply is configured for providing electric power for electric cauterization. The system further comprises a magazine comprising the tool set and a data-processing system. The surgery device comprises a body element, a patient component comprising a distal portion, and a toolset interface. The toolset interface is configured for receiving a magazine comprising the tool set. The surgery device further comprises a tool activation system. The tool activation system is configured for mounting at least one tool of the tool set to the distal portion of the patient component. The surgery device further comprises an operating element. The distal portion of the patient component is configured to be introduced into a body of a patient. The magazine is configured to be mounted to the toolset interface of the surgery device.

## Description

The present invention relates to the field of surgery devices, particularly to the field of microinvasive surgery devices.

WO 02/065933 discloses a remotely controllable surgical instrument comprising an instrument shaft having a proximal end drivably intercoupled to a drive unit and a distal end insertable within a subject for performing a medical procedure at an operative site within the subject, a remotely disposed user interface interconnected to a remotely disposed signal processor which processes commands received from the user interface, the signal processor interconnected to the drive unit for transmitting processed command signals received from the user interface to the drive unit characterized in that the instrument shaft comprises an elongated disposable shaft removably interconnected to the drive unit via a coupling mechanism and a distal instrument drivable via cables.

US 2007/0239186 A1 discloses a method of performing a medical procedure (e.g., a cardiac bypass procedure) on a patient. The method comprises introducing at least one medical instrument into a patient (e.g., percutaneously), conveying control signals from a remote controller to a drive unit, and operating the drive unit in accordance with the control signals to actuate at least one tool respectively located on the medical instrument(s) to transversely secure a first anatomical vessel (e.g., a blood vessel) to a sidewall of a second anatomical vessel (e.g., another blood vessel). In one method, the control signals are conveyed from the remote controller to the drive unit in response to user commands. The user commands may be movements made at a user interface that correspond to movements of the medical instrument(s).

US 2018/0116735 A1 discloses robotic surgical tools, systems, and methods for preparing for and performing robotic surgery include a memory mounted on the tool. The memory can perform a number of functions when the tool is loaded on the tool manipulator: first, the memory can provide a signal verifying that the tool is compatible with that particular robotic system. Secondly, the tool memory may identify the tool-type to the robotic system so that the robotic system can reconfigure its programming. Thirdly, the memory of the tool may indicate tool-specific information, including measured calibration offsets indicating misalignment of the tool drive system, tool life data, or the like. This information may be stored in a read only memory (ROM), or in a nonvolatile memory which can be written to only a single time. The invention further provides improved engagement structures for coupling robotic surgical tools with manipulator structures.

US 9763725 B2 discloses a system for tracking use of a medical device includes an electrosurgical generator, a readable module and a read module. The electrosurgical generator is configured to selectively deliver an electrosurgical energy signal to an electrosurgical delivery device connected to the electrosurgical generator. The readable module is connected to the electrosurgical delivery device and configured to uniquely identify the electrosurgical delivery device. The read module is in communication with the electrosurgical generator that identifies the read module, the read module configured to identify the readable module and further configured to determine the viability of the electrosurgical delivery device. Delivery of electrosurgical energy to the electrosurgical delivery device is enabled by the read module if the electrosurgical delivery device is a viable device.

US 11801102 B2 discloses robotic devices, systems, and methods for use in robotic surgery and other robotic applications, and/or medical instrument devices, systems, and methods includes both a reusable processor and a limited-use robotic tool or medical treatment probe. A memory the limited-use component includes machine readable code with data and/or programming instructions to be implemented by the processor. Programming of the processor can be updated by shipping of new data once downloaded by the processor from a component, subsequent components can take advantage of the updated processor without repeated downloading.

US 11638587 B2 discloses a surgical device for applying clips is disclosed including a cartridge, an RFID tag, and a controller. The cartridge includes a plurality of clips. A crimping drive is configured to move a first jaw and a second jaw to a closed position during a crimping stroke. One of the plurality of clips is crimped around tissue during the crimping stroke. Stored data on the RFID tag relates to an identifying characteristic of at least one of the plurality of clips within the cartridge. An RFID scanner is configured to receive a first signal from the RFID tag in response to an interrogation signal. The first signal includes the stored data on the RFID tag. A controller in communication with the RFID scanner is configured to compare the stored data to a set of compatibility data and vary an operational parameter of the surgical device based on the stored data.

US 11653920 B2 a surgical instrument system that comprises a shaft and a handle assembly releasably couplable to the shaft. The handle assembly comprises a disposable outer housing defining a sterile barrier. The disposable outer housing is movable between an open configuration and a closed configuration. The handle assembly further comprises a control inner core receivable inside the disposable outer housing in the open configuration. The disposable outer housing is configured to isolate the control inner core within the sterile barrier in the closed configuration. The surgical instrument system further comprises an end effector releasably couplable to the shaft and an electrical interface assembly configured to transmit data and power between the control inner core and the end effector. The electrical interface assembly comprises a first interface portion on a first side of the sterile barrier, and a second interface portion on a second side of the sterile barrier opposite the first side.

EP24151941.2, which is assigned to the applicant of the present application, and which is incorporated by reference in its entirety, discloses: A microinvasive surgery device comprising a body element; a plurality of tools; a tube component having a proximal portion and a distal portion, wherein the proximal portion of the tube component is connected to the body element; a magazine configured for receiving the plurality of tools; a tool activation mechanism, wherein the tool activation mechanism is configured for mounting one of the plurality of tools to the distal portion of the tube component; and an operating element. The magazine is located at the body element. The tool activation mechanism is configured for interchanging the tool mounted to the distal portion of the tube component. The operating element is an operating lever. The microinvasive surgery device is a handheld device. Further disclosed are different methods comprising using the microinvasive surgery device for microinvasive surgery and/or connecting the microinvasive surgery device to a generator configured for providing electric power for electric cauterization.

While the prior art approaches may be satisfactory in some regards, they have certain shortcomings and disadvantages.

It is therefore an object of the invention to overcome or at least alleviate the shortcomings and disadvantages of the prior art. More particularly, it is an object of the present invention to provide an improved surgery system and a method for use thereof.

It is another optional objective of the present invention to provide an improved microinvasive surgery system and device allowing and a method for use of the system and/or the device.

It is another optional objective of the present invention to provide a microinvasive surgery system enabling more safe and reliable usage of different operating tools and a method for use of the system.

In a first embodiment, a method is disclosed. The method comprises using a surgery device. The surgery device comprises a patient component, such as a tube component. The surgery device further comprises a tool activation system and a toolset interface.

The method further comprises using a data-processing system and a tool set comprising a plurality of tools.

The patient component, particularly a distal end of the patient component, may be configured to be inserted into a body of a patient.

The term "distal" may refer to a side of the surgery device facing a patient when a tool of the surgery device and/or the patient component is inserted into the body of the patient and/or a direction towards this side. The term "proximal" may refer to a side of the surgery device opposite to the side facing the patient and/or to a corresponding direction.

The plurality of tools may for example comprise a scissors tool, a probe, a dissector, a hook and/or a grasper.

The method may comprise mounting the tool set to the toolset interface.

The method may comprise using a magazine comprising the tool set. Mounting the tool set to the toolset interface may be mounting the magazine to the toolset interface.

The toolset interface may be a mechanical interface of the surgery device configured for receiving the tool set or the magazine. The mechanical interface may for example comprise a rotatable coupling to receive a magazine in the which the tools are arranged in a revolver-drum-like shape.

The method may comprise activating at least one of the tools. Activating the at least one tool may comprise mounting the at least one tool to the distal portion of the patient component by the tool activation system. The terms "activating a tool" and "mounting a tool" may thus be intended to be synonyms in the present disclosure. An activated tool may thus be a tool mounted to the distal portion of the patient component by the tool activation system.

The surgery device may comprise a motor system. The magazine may comprise a plurality of receiving locations, such as a plurality of chambers. For example, each receiving location may be configured for receiving one or at least one tool.

Activating the at least one tool may comprise aligning one of the receiving locations with the tool activation mechanism by the motor system, for example a receiving location holding said tool.

The method may comprise the motor system rotating the magazine about a rotation axis when mounted to the toolset interface.

The patient component may be the tube component. Aligning the one of the receiving locations with the tool activation mechanism by the motor system may be aligning said receiving location with the patient component, such as with the tube component. Thus, optionally advantageously, the tool may be moved from the magazine along the patient component in order to be activated.

Mounting the tool set, particularly the magazine, to the toolset interface may comprise opening a packaging of the tool set, particularly of the magazine. Thus, optionally advantageously, hygienic and/or tamper-proof handling of the tool set may be facilitated.

The method may comprise processing toolset information data relating to the tool set, particularly to the tool set mounted to the toolset interface.

The toolset information data may relate to the tool set that the magazine comprises.

The toolset information data may comprise an indication of a type of each tool of the toolset.

The toolset information data may comprise an indication of a functionality of each tool of the toolset.

The toolset information data may comprise, for each tool of the tool set, an indication of a receiving location containing the respective tool. The receiving location may for example be specified with respect to the toolset interface, e.g., in case of a drum-like magazine, as angular offset to a rotatable portion of the toolset interface configured to rotate with the magazine.

Processing the toolset information data may comprise processing a toolset identifier.

The method may comprise determining the toolset identifier based on an identification element associated with the tool set.

At least one of the packaging and the magazine may comprise the identification element.

The data-processing system may comprise a data-exchange component. Determining the toolset identifier based on the identification element may comprise reading the toolset identifier from the identification element by means of the data-exchange component.

The data-processing system may comprise at least one component separate from the surgery device, for example in a separate box or console and/or in a power supply for the surgery device.

The data-processing system may however also comprise at least one component of the surgery device. For example, the surgery device may comprise the data-exchange component or a data-exchange unit of the data-exchange component. However, the data-exchange component or a data-exchange unit thereof may also be separate from the surgery device, e.g., in a non-sterile environment.

Processing the toolset identifier may comprise the data-processing system receiving data relating to the toolset information data from a server. For example, processing the toolset identifier may comprise as sending the toolset identifier to the server and receiving the toolset information data from the server, or receiving data listing toolset identifiers and corresponding toolset information data from the server and determining the toolset information data of the tool set based thereon.

The method may comprise generating alignment data. The alignment data may be indicative of the receiving location aligned with the tool activation mechanism, particularly the receiving location aligned with the patient component.

For example, the alignment data may be indicative of a rotational position of the magazine relative to the patient component, such as the tube component, e.g. a rotation angle. The alignment data may, additionally or alternatively, comprise an ordinal number or indication of the receiving location aligned with the tube component.

Generating the alignment data may comprise determining a position and/or an orientation of the magazine with respect to the tool activation system, particularly with respect to the patient component.

Generating the alignment data may comprise sensing a rotational position of the magazine with respect to the tool activation mechanism, particularly with respect to the patient component. This may be in line with the above-discussed example.

Generating the alignment data may comprise sensing a rotational position and/or an orientation of the motor system.

This may for example apply in embodiments wherein the tool interface may be configured for receiving a magazine only with a predetermined angular offset vis-à-vis a coupling of the motor system. In such embodiments, at a given position and/or orientation of a magazine-side of the motor system, such as a given rotational position, only one orientation of the magazine may be possible.

Sensing the rotational position of the magazine and/or the rotational position of the motor system may comprise using at least one of
- a rotary encoder,
- a plurality of magnets and at least one sensor selected from a hall sensor and/or a reed switch,
- a mechanical switch configured for engaging with a part of the magazine, such as an indentation or a protrusion of the magazine or a part of the motor system,
- an optical rotation sensor, and/or
- an RFID-element and a corresponding reader.

Suitable arrangements of these components are further discussed below with reference to the surgery device/a system comprising the surgery device.

The method may comprise processing receiving-location-data for at least one of the activated tool of the tool set and the receiving location aligned with the tool activation mechanism.

The receiving-location-data may indicate a suitability for electro-cauterization of said activated tool or a tool associated with the receiving location aligned with the tool activation mechanism.

In the present disclosure, the phrase "the receiving-location-data may indicate a suitability for electro-cauterization" is intended to specify that the receiving-location-data are used to encode such a suitability.

The receiving-location-data may for example indicate whether a tool is suitable for electro-cauterization or not suitable for electro-cauterization. The receiving-location-data may also indicate whether a tool is suitable for monopolar electro-cauterization, for bipolar electro-cauterization or not suitable for electro-cauterization.

For example, tools associated with certain receiving locations of a magazine may be suitable to electro-cauterization of a certain type, e.g., monopolar electro-cauterization. In other words, in some embodiments, certain receiving locations may only comprise tools of a certain type.

In some embodiments, the receiving-location-data may at least indicate whether a tool is suitable for monopolar electro-cauterization or not. This may optionally advantageously avoid provision of power for monopolar electro-cauterization with tools that are not suitable for monopolar electro-cauterization, which might otherwise result in injuries of the patient. Hence, optionally advantageously, a safety of the method may thus be increased.

Processing the receiving-location-data may comprise sensing, by a remainder of the surgery device, e.g., the data-exchange component, a receiving location element associated with the receiving location aligned with the patient component and/or a presence of a receiving location element associated with the receiving location aligned with the patient component. In other words, a receiving location element associated with a receiving location of a currently activated tool or a tool to be activated may be sensed for processing, particularly for generating the receiving-location-data.

The receiving location element may for example be a portion of the magazine or an element mounted to the magazine. In particular, the receiving location element may be mounted next to or with a pre-defined offset to the respective receiving location.

The method may comprise generating the receiving-location-data based on the sensed receiving location element.

In other words, the receiving-location-data may relate to a receiving location element associated with a receiving location of a tool.

The receiving location element and a corresponding sensor associated with a remainder of the surgery device may be selected from
- a plurality of magnets and at least one sensor selected from a hall sensor and/or a reed switch associated with the remainder of the surgery device,
- a portion of the magazine comprising a protrusion or indentation of the magazine and a mechanical switch associated with the remainder of the surgery device, which mechanical switch is configured for engaging with said portion of the magazine,
- a marking on the magazine configured to be read by an optical rotation sensor associated with the remainder of the surgery device, and/or
- an RFID-element and a corresponding reader associated with a remainder of the surgery device.

In the example of the marking and/or the protrusion or indentation, such marking and/or protrusion or indentation may be located, for example, next to the respective receiving location.

In another example, each receiving location of the magazine comprising a tool for monopolar electro-cauterization may comprise a magnet, thus optionally advantageously allowing for improved reliability of a detection of receiving locations associated with tools suitable for monopolar electro-cauterization, hence optionally advantageously improving safety of the method for the patient.

The method may comprise the sensor comprising a switching component. In other words, the sensor may comprise the switching component. The switching component may inhibit provision of power to the tool based on the sensed receiving location element.

In other words, if a receiving location element of a first type is sensed, the switching component may not inhibit provision of power to the tool based on the sensed receiving location element. If a receiving location element of a second type is sensed, or if no receiving location element is sensed, the switching component may inhibit provision of power to the tool.

The switching component may for example be a separate switch, such as a mechanical switch or a relay triggered by the sensor associated with the remainder of the surgery device.

The receiving-location-data may indicate at least whether a tool is configured
(a) for monopolar electro-cauterization, or
(b) for at least one of bipolar electro-cauterization and/or no electro-cauterization.

As set out above, erroneous provision of power for monopolar electro-cauterization may be dangerous for a patient. Thus, optionally advantageously, such receiving-location-data may provide for improved safety of the method.

In the present disclosure, the term "a tool is configured for monopolar electro-cauterization" is intended to be equivalent to "the tool is suitable for monopolar electro-cauterization". "Suitable for monopolar electro-cauterization" is intended to mean that such a tool can be used for monopolar electro-cauterization in a safe manner, as other tools intended for monopolar electro-cauterization. The terms "a tool is configured for bipolar electro-cauterization" and "the tool is suitable for bipolar electro-cauterization" are intended to be interpreted analogously.

The receiving-location-data may indicate at least whether a tool is configured
(a) for monopolar electro-cauterization,
(b) for bipolar electro-cauterization, or
(c) not configured for electro-cauterization.

Thus, optionally advantageously, automated control of provision of power when using a device, such as the power supply, which is configured for providing power for monopolar and bipolar electro-cauterization may be enabled. Hence, optionally advantageously, a safe and efficient use of such a device or system may be facilitated.

The receiving-location-data may indicate at least whether a tool is
(a) configured for electro-cauterization, or
(b) not configured for electro-cauterization.

This may optionally advantageously avoid erroneous operation of tools that are not configured for electro-cauterization and thus provide for a more efficient and/or safe method.

The method may comprise processing tool type data, wherein the tool type data relate to the at least one tool mounted to the distal portion of the patient component.

The method may comprise generating the tool type data based on a tool data element. Each tool may comprise a tool data element.

Each tool data element may be selected from at least one of a geometrical element of the tool, a magnetic portion of the tool, an optical marking of the tool, an RFID-element of the tool and a resistance of the tool.

The tool data elements may be of a same type. In other words, each tool may comprise a tool data element of a same type of the above list, however, the person skilled in in the art will easily understand that the tool data elements may indicate different tool type data for at least two tools.

Generating the tool type data may comprise sensing the resistance of the activated tool. Sensing the resistance may be performed by known means of measuring an electric resistance.

Processing the tool type data may comprise determining the tool type data relating to the at least one tool mounted to the distal portion of the patient component based on the toolset information data and the alignment data.

The method may comprise outputting device data.

The device data may for example comprise an indication of the tool type data. In other words, the device data may, for example, comprise an indication of types of tools of the tool set, and/or a type of the activated tool.

The device data may further comprise an indication of the receiving-location-data. Thus, optionally advantageously, errors when activating electro-cauterization by a user for a tool not configured for electro-cauterization may be reduced and/or avoided.

The device data may further comprise an indication of a status of the surgery device, for example whether the surgery device is correctly assembled. Such status data may for example be generated by means of assembly switches mounted to components of the surgery device.

The method may comprise using a camera and a camera screen outputting image data captured by the camera. The image data may relate to an inside of a body of the patient.

In other words, the method may comprise using a camera and a screen for laparoscopic and/or endoscopic surgery.

In such embodiments, outputting the device data, particularly the indication of the tool type data, may comprise outputting the indication of the tool type data together with the image data generated by the camera by means of the camera screen. For example, the indication of the tool type data may be outputted as an overlay or side-by-side with the camera image data generated by the camera via the camera screen.

Thus, optionally advantageously, the activated tool may be easily verified by the user during a surgery, particularly during a microinvasive surgery.

In another embodiment, outputting the indication of the tool type data may comprise outputting the indication of the tool type data by means of an additional screen connected to the data-processing system. Thus, optionally advantageously, integration in an existing system for microinvasive surgery may be facilitated, as fewer modifications may be necessary.

The surgery device may comprise an optical output element, such as an LCD-screen or a plurality of LEDs mounted to the surgery device. The method may comprise electrically controlling the optical output element to output an indication of the tool type data.

The optical output element may be a digital output element. In other words, the optical output element may generate a discrete output based on a digital input.

The surgery device may comprise a mechanical output element, such as a plurality of moveable optical markers. The mechanical output element may be mechanically coupled to the magazine in a mounted configuration. The method may comprise outputting an indication of the tool type data by means of the mechanical output element.

The method may comprise outputting an acoustic indication of the tool type data, e.g. by means of a speaker.

The method may comprise providing power for electro-cauterization to at least one first tool of the tool set by means of the power supply. The method may comprise not providing power for electro-cauterization to at least one second tool of the tool set.

The method may comprise providing power for monopolar electro-cauterization to at least one tool of the tool set, particularly by means of the power supply, and not providing power for monopolar electro-cauterization to still at least one other tool of the tool set.

Particularly, the method may comprise at least two surgery modes. The surgery modes may comprise a surgery mode for monopolar electro-cauterization, such as a surgery mode for monopolar electro-cauterization and a surgery mode without electro-cauterization.

The term "surgery mode" is intended to refer to a mode of operating of the surgery device, the power supply and/or other components relating to provision of power for electro-cauterization to a patient.

The term "activating" a surgery mode is intended to specify that the surgery device, the power supply and/or other components assume an operating mode suitable to provide power for electro-cauterization according to the activated surgery mode. However, an additional command or trigger to provide power, such as a user input, may be required for provision of the power despite activation of the surgery mode.

The method may comprise providing power for monopolar electro-cauterization to at least one tool of the tool set, power for bipolar electro-cauterization to at least one other tool of the tool set, particularly by means of the power supply, and not providing power for electro-cauterization to still at least one other tool of the tool set.

The surgery modes of may comprise
- a surgery mode for monopolar electro-cauterization,
- a surgery mode for bipolar electro-cauterization, and
- a surgery mode without cauterization.

The device data may comprise an indication of a current surgery mode to be activated, such as a surgery mode corresponding to the activated tool.

The method may comprise generating electro-cauterization instruction data. The method may further comprise the power supply providing power for electro-cauterization to the at least one first tool based on the electro-cauterization instruction data.

The method may comprise generating the electro-cauterization instruction data. The method may comprise providing power for electro-cauterization according to the surgery mode activated based on the electro-cauterization instruction data.

The method may comprise operating one or more relays, electronic switching units, such as power transistors, a toggle switch or the like to provide a connection of a suitable portion of the power supply and the activated tool. For example, these may be located in the surgery device, the console, an outer housing of the power supply and/or with the data-processing system.

The suitable portion of the power supply may for example be one or more outputs for power for electro-cauterization. In this example, the method may comprise providing connection between the tool and the output for monopolar electro-cauterization at some point in time or time interval, the output for bipolar electro-cauterization at some other point in time or time interval, and not providing a connection to these two outputs at still another point in time or time interval.

Additionally or alternatively, the method may comprise operating one or more relays, electronic switching units, such as power transistors, a toggle switch or the like to provide a connection of a suitable portion of the power supply and an activation element, such as a button of the surgery device configured to receive a user input to start electro-cauterization. For example, the method may comprise providing connection of the activation element and a command receiving port for monopolar electro-cauterization of the power supply, a command receiving port for bipolar electro-cauterization and none of the command receiving ports at different points in time or time intervals.

The method may comprise generating the electro-cauterization instruction data at least based on the toolset information data and the alignment data.

In other words, the method may comprise generating the electro-cauterization instruction data based on the tools of tool set, particularly of the magazine, and a position of magazine and/or a receiving location aligned with the patient component.

For example, the method may comprise reading information about which type of tool is associated with which receiving location, e.g., from a code associated with the magazine or its packaging. The method may then comprise, e.g., the data-processing system receiving information about the magazine position from the sensor, and the data-processing system may combine the information and generate corresponding electro-cauterization instruction data.

The method may comprise generating the electro-cauterization instruction data at least based on the receiving-location-data. Thus, optionally advantageously, correct control of the power supply may be facilitated and safety of the method may be improved.

For example, the method may comprise generating the electro-cauterization instruction data based at least on the receiving-location-data, which may be generated based on the receiving location element. In other words, the magazine may comprise receiving location elements configured for marking individual receiving locations, such as chambers, of tools. The receiving location elements may, in the example, be indicative of the suitability for electro-cauterization of the tool contained by the receiving location. The presence, type or data from the receiving location elements may be read by the system, e.g., the data-exchange component, the surgery device and/or another sensor of a remainder of the system.

The method may comprise generating the electro-cauterization instruction data at least based on the alignment data.

The magazine may comprise
- a first set of receiving locations of the magazine only comprising one or more tools for monopolar electro-cauterization, and
- a second set of receiving locations of the magazine only comprising one or more tools for bipolar electro-cauterization and/or for no electro-cauterization.

Each set of receiving locations of the magazine may be arranged at pre-determined regions of the magazine.

The regions may however be discontinuous, e.g., in case of six receiving locations 1-6 that are arranged circularly at the magazine, the first set may comprise receiving locations 1, 2 and 5 and the second set may comprise receiving locations 3 and 4. Receiving location 6 may or may not be comprised by one of the sets.

In some embodiments, the first set of receiving locations of the magazine may only comprise one or more tools for monopolar electro-cauterization, and the second set of receiving locations of the magazine may only comprise one or more tools for bipolar electro-cauterization. A third set of receiving locations may only comprise one or more tools not intended or not configured for electro-cauterization.

The method may comprise generating the electro-cauterization instruction data at least based on the tool type data generated based on the tool data element of the activated tool.

In other words, in line with this example, each tool may have a tool data element indicative of its electro-cauterization suitability/surgery mode. The tool data element may then be read by the system, the data-exchange component, and the system, e.g., the switching component and/or the data-exchange component, may then generate the electro-cauterization instruction data. However, the switching component may also additionally inhibit provision of power if the tool data element indicates that the tool is not configured for electro-cauterization or not configured for an activated mode of electro-cauterization.

For example, the switching component may be configured for interrupting the connection of the suitable portion of the power supply and the activated tool and/or the activation element when inhibiting the provision of power.

The surgery mode for monopolar electro-cauterization may be activated based on the toolset information data and the alignment data. In particular, the surgery mode for electro-cauterization may be activated based on the tool type data generated based on the toolset information data and the alignment data, and further only if the receiving-location-data corresponding to an activated tool indicate that the mounted tool is configured for monopolar electro-cauterization.

In other words, in such embodiments, even if the toolset information data and the alignment data indicate that a tool for monopolar electro-cauterization is activated/aligned with the tool activation system, the surgery mode for monopolar electro-cauterization may not be activated if the receiving-location-data do not indicate that the mounted tool is suitable for monopolar electro-cauterization.

Optionally advantageously, activating the surgery mode for monopolar electro-cauterization based on the toolset information data, the alignment data and the receiving-location-data, and activating the surgery mode for bipolar electro-cauterization and no electro-cauterization based on the toolset information data and the alignment data, may provide for a good trade-off between safety of the method, as the surgery mode for monopolar electro-cauterization is not erroneously activated, and flexibility, as the tools for bipolar electro-cauterization and no electro-cauterization may be activated based on the toolset information data and the alignment data.

In other words, in some embodiments, the surgery mode may be selected based on the tool type data, e.g., by processing the alignment data and the toolset information data, but monopolar electro-cauterization may be inhibited unless a matching receiving location element or tool data element is detected. Thus, optionally advantageously, a practicable trade-off between safety and flexibility of the method may be provided.

The surgery modes for bipolar electro-cauterization and no electro-cauterization may be activated based on the toolset information data and the alignment data, particularly based on the tool type data generated based on the toolset information data and the alignment data.

The step of only activating the surgery mode for monopolar electro-cauterization if the receiving-location-data corresponding to an activated tool indicate that the mounted tool is configured for monopolar electro-cauterization may comprise the switching component inhibiting activation of the surgery mode for monopolar electro-cauterization and/or inhibiting power transmission from the power supply to the at least one tool.

Thus, optionally advantageously, safety may further be increased, as the switching component may inhibit accidental or faulty provision of power for monopolar electro-cauterization, i.e., accidental or faulty activation of the surgery mode for monopolar electro-cauterization, even in case of a malfunction of another component or wrong toolset information data.

As set out above, the switching component may for example comprise a mechanical switch or a relay configured to inhibit provision of power or power for monopolar electro-cauterization.

In some embodiments, the surgery mode for monopolar electro-cauterization may only be activated if a tool from the first set of receiving locations is activated. In such embodiments, the surgery modes for bipolar electro-cauterization and no electro-cauterization may be activated based on the toolset information data and the alignment data, particularly based on the tool type data generated based on the toolset information data and the alignment data. Thus, optionally advantageously, no receiving location elements are necessary, making the magazine less complex.

The method may comprise, upon a begin of bipolar electro-cauterization, determining whether the tool mounted to the distal portion of the patient component is currently suitable for bipolar electro-cauterization based on at least one electrical property of said tool, such as a resistance and/or an impedance of the tool mounted to the distal portion of the patient component. In particular, the power supply may determine upon the begin of bipolar electro-cauterization whether the tool mounted to the distal portion of the patient component is currently suitable for bipolar electro-cauterization.

For example, in case of a tool for monopolar electro-cauterization, a tool that is not intended for electro-cauterization, and/or a short-circuited tool for bipolar electro-cauterization, it may be determined that the respective tool is currently unsuitable for bipolar electro-cauterization based on the respective tool's resistance and/or the impedance.

The person skilled in the art will easily understand that a tool that is generally suitable or configured for bipolar electro-cauterization may at a given point in time not be suitable for bipolar electro-cauterization, e.g., if it is short-circuited, as in the case of a closed pincers tool.

The method may comprise preventing a movement of the receiving locations, particularly of the magazine, with respect to the tool activation mechanism while a tool is mounted to the distal portion of the patient component.

As disclosed in the above-mentioned application EP24151941.2, this may, e.g., be achieved by appropriate control of the motor system, by use of pins and/or a lockable transmission of the motor system, and/or by a suitable mechanical design of the tool activation system.

The method may comprise storing settings for electro-cauterization for at least one of the tools for monopolar electro-cauterization and/or at least one of the tools for bipolar electro-cauterization.

For example, for each of the tools, a specific high-frequency electro-cauterization mode, a high-frequency intensity, a high-frequency duty cycle and/or a high-frequency waveform may be stored.

Thus, optionally advantageously, the settings may be specific to each tool and not only to each tool type or type of electro-cauterization.

At least one of the settings may relate to a single tool.

At least one of the settings may relate to all tools for monopolar/bipolar electro-cauterization.

The method may comprise receiving the settings for electro-cauterization by a user input, such as a voice input and/or a button interface of the surgery device. For example, a button may trigger an activation of the voice input.

The method may comprise the power supply providing power to the tools based on the stored settings.

The surgery device may be a microinvasive surgery device.

The method may comprise receiving at least one tool selection input from a user, the tool selection input being indicative of a tool to be mounted.

The method may comprise receiving the at least one tool selection input by means of a voice input, a button interface of the surgery device and/or a control interface for a robotic surgery system.

The method may comprise, in response to receiving the tool selection input, mounting a selected tool.

The method may comprise generating a tool suggestion and outputting the tool suggestions.

Generating the tool suggestion may be based on tool use data relating to a use of tools and/or a use of the surgery device.

The surgery device may be a robotic surgery device, particularly a robotic microinvasive surgery device.

The operating element may be robotically actuated.

The surgery device may be a manually operated device and/or a handheld device, particularly a manually operated microinvasive surgery device and/or a handheld microinvasive surgery device.

Also disclosed are a surgery device and a system. Advantages and details discussed in the context of the method may respectively apply also in the context of the surgery device as well as the system.

In a second embodiment, a surgery device is disclosed.

The surgery device comprises the body element, the patient component comprising the distal portion and the toolset interface. The toolset interface is configured for receiving the tool set comprising the plurality of tools. The surgery device further comprises the tool activation system. The tool activation system is configured for mounting at least one tool of the tool set to the distal portion of the patient component. The surgery device further comprises an operating element.

The surgery device may be a handheld surgery device or a robotic surgery device. The body element may thus be the body element of the handheld device, or the body element of the robot surgery device.

The patient component may also be the tube component. The distal portion of the patient component, particularly of the tube component, may be configured to be introduced into the body of the patient.

The operating element may be an operating lever, e.g. for operating a tool mounted to the surgery device.

The patient component may be the tube component comprising a proximal portion and the distal portion. The proximal portion of the tube component may be connected to the body element.

The toolset interface may be configured for receiving the magazine. The magazine may comprise the tool set. In particular, the magazine may be configured for receiving the plurality of tools of the set at a same time.

The tool activation system may be configured for activating at least one tool of the tool set by mounting the at least one tool to the distal portion of the patient component.

The tool activation system may be configured for moving the at least one tool from the magazine, particularly for mounting the at least one tool to the distal portion of the patient component.

The tool activation system may be configured for deactivating the at least one tool by unmounting the at least one tool from the distal portion of the patient component.

Unmounting the at least one tool may comprise moving the at least one tool to the magazine.

The tool activation system may be configured for interchanging the tool mounted to the distal portion of the patient component, particularly while the distal portion of the patient component is in the surgery configuration.

In the surgery configuration, the distal portion of the patient component may be introduced into the body of the patient.

The tool activation system may comprise a tool coupling element configured for releasably coupling one of the tools to the tool activation system, particularly for releasably coupling one tool at a time to the tool activation system.

The tool coupling element may be configured for being coupled to the tool and for being decoupled from the tool in a magazine configuration.

The magazine configuration may be a configuration in which the tool coupling element is located in and/or adjacent to the magazine.

The tool coupling element may be configured for being moved along an inside of the tube component from the magazine configuration towards the distal portion of the tube component. The tool coupling element may be in the mounted configuration when the tool is mounted to the distal portion of the tube component.

The body element may comprise the toolset interface.

The surgery device may comprise the motor system. The motor system may be configured for moving the tool set coupled to the toolset interface with respect to the body element, particularly for moving the magazine coupled to the body element with respect to the body element.

The motor system may be configured for rotating the magazine about the rotation axis when mounted to the toolset interface.

The toolset interface may be configured for receiving the magazine comprising the plurality of tool receiving locations. Each tool receiving location may be configured for receiving at least one tool. The motor system may be configured for moving the magazine in the toolset interface so as to align one tool receiving location at a time with the patient component.

In particular, the motor system may be configured for moving the magazine in the toolset interface in a configuration where the toolset interface receives the magazine.

For example, the surgery device may be configured for aligning a tool receiving location with the patient component for activating a tool which is received in the receiving location.

The surgery device may be configured for sensing the position and/or the orientation of the magazine with respect to the tool activation system, particularly with respect to the patient component.

The surgery device is configured for sensing the rotational position of the magazine with respect to the tool activation mechanism, particularly with respect to the patient component.

The surgery device may be configured for sensing a rotational position and/or an orientation of the motor system 122.

For example, the tool interface may be configured for receiving a magazine only with a predetermined angular offset vis-à-vis a coupling of the motor system. In other words, in the example, at a given position/orientation of a magazine-side of the motor system, such as an output shaft of the motor system, only one orientation of the magazine is possible.

The surgery device may comprise at least one magazine sensor selected from
- the hall sensor and/or the reed switch,
- the mechanical switch,
- the optical rotation sensor, and/or
- the RFID-reader.

The magazine sensor may be configured for sensing the rotational position of the magazine with respect to the tool activation mechanism, particularly with respect to the patient component.

The surgery device may comprise at least one motor sensor selected from
- a rotary encoder,
- a hall sensor and/or a reed switch,
- a mechanical switch,
- an optical rotation sensor, and/or
- an RFID-reader.

The motor sensor may be configured for sensing the rotational position of the motor system.

In embodiments where at least one motor sensor is a hall sensor and/or a reed switch, the motor system, e.g. the output shaft of the motor system, may comprise at least one magnetic marker to mark a pre-determined orientation of the output shaft.

In embodiments where at least one motor sensor is a mechanical switch, the motor system, e.g. the output shaft, may comprise at least one protrusion and/or at least one indentation.

In embodiments where at least one motor sensor is an optical sensor, the motor system, e.g., the output shaft, may comprise corresponding optical markings.

Further, in cases where at least one motor sensor is an RFID-reader, the motor system may comprise corresponding RFID-element.

The surgery device may be configured for preventing the movement of the receiving locations, particularly of the magazine, with respect to the tool activation mechanism while a tool is mounted to the distal portion of the patient component.

As disclosed in the above-mentioned application EP24151941.2, this may, e.g., be achieved by appropriate control of the motor system, by use of pins and/or a lockable transmission of the motor system, and/or by a suitable mechanical design of the tool activation system.

The operating element may be the operating lever. The operating element may be configured for mechanically actuating the activated tool.

The tool activation system may comprise a rack and/or a rod.

The rack and/or the rod may be configured for transferring tension and compression to the mounted tool by means of the tool coupling element.

The tool activation system may be configured for moving the at least one tool from the magazine to the distal portion of the tube component by moving the tool along the inside of the tube component using the rack.

The tool activation system may further be configured for moving the at least one tool from the distal portion of the tube component to the magazine by moving the tool along the tube inside of the tube component using the rack.

A distal end of the rack may be connected to the tool coupling element. The tool activation system may be configured for moving the tool coupling element from a magazine configuration to a mounted configuration by means of the rack.

The tool coupling element may be configured for coupling the tool to the rack.

The tube component may be configured for transmitting electric power for electro-cauterization to at least one mounted tool.

The body element may comprise the bore, such as a sleeve bearing, configured for receiving the tube component. The bore may be made from a conductive material, such as stainless steel. The bore may be configured for transmitting electric power from the body element to the tube component.

The tube component may comprise an electrically insulating outer layer and an electrically conductive layer within the insulating outer layer.

The tube component may comprise a cable configured for conducting electric power from the body element to the at least one mounted tool.

At least a section of the rod may be configured for transmitting electric power to the at least one tool.

The motor system may be configured for moving the tool coupling element between the magazine configuration and the mounted configuration.

The motor system may comprise a rack drive. The rack drive may comprise a gear engaging with the rack.

The rack drive may comprise a rack-drive-motor and a rack-drive-transmission. The rack-drive-motor may be connected to the rack-drive-transmission. The rack-drive-transmission may be self-locking, particularly not backdrivable.

The rack-drive-transmission may comprise a self-locking worm-gear unit.

The rack-drive-transmission may comprise an automatic locking mechanism.

The microinvasive surgery device, particularly the motor system, may comprise a magazine drive configured for moving the magazine so as to assume a position of the plurality of positions corresponding to the receiving locations.

The motor system may comprise a gear stage, such as a planetary gear, configured to enable moving the tool set coupled to the toolset interface with respect to the body element.

The surgery device may comprise the input interface, such as the button interface.

The surgery device may be a microinvasive surgery device.

The surgery device may be the robotic surgery device, particularly the robotic microinvasive surgery device.

The operating element may be robotically actuated.

However, the surgery device may also be the manually operated device and/or the handheld device, particularly the manually operated microinvasive surgery device and/or the handheld microinvasive surgery device.

The surgery device may be configured for performing the method according to any of the method embodiments.

The method may comprise using the above-disclosed surgery device.

In a third embodiment, a system is disclosed. The system comprises the surgery device, the tool set comprising the plurality of tools, the data-processing system and the power supply. The power supply is configured for providing electric power for electric cauterization.

The system may further comprise a user-interface device.

The user-interface device may comprise the camera screen configured for outputting the image data generated by the camera for microinvasive surgery. For example, the image data may relate to an inside of a body of the patient.

The user-interface device may further comprise an additional screen. The additional screen may be connected to the data-processing system.

The system may comprise the camera for microinvasive surgery.

The data-processing system may comprise the data-exchange component.

The system may comprise the magazine comprising the tool set.

The tool set, particularly the magazine, may be configured for being mounted to the toolset interface of the surgery device.

The magazine may comprise the plurality of receiving locations, such as a plurality of chambers. Each receiving location may be configured for receiving one or at least one tool.

Activating the at least one tool may comprise aligning one of the receiving locations with the tool activation mechanism, particularly with the patient component. The motor system may be configured for aligning the one of the receiving locations with the tool activation mechanism, particularly with the patient component.

The tool set, particularly the magazine, may comprise the packaging.

The packaging may be a sterile packaging, such as a sterile bag.

The magazine may be configured to be mounted to the toolset interface of the surgery device.

At least one tool of the tool set may be a multi-part-tool. The at least one multi-part-tool may comprise at least one movable tool part. The at least one moveable tool part may be configured for being moved relative to another part of the tool, such as a remainder of the tool or a second moveable part of the tool.

The at least one multi-part-tool may comprise a scissors tool and/or a forceps tool. In other words, the toolset may comprise at least one multi-part-scissors tool and/or at least one multi-part-forceps tool.

The at least one movable tool part may be movable by an application of force when the multi-part-tool is in a mounted configuration.

The tools of the tool set may be tools for microinvasive surgery.

At least one tool of the tool set may be the tool for electro-cauterization.

The at least one multi-part-tool may comprise at least one multi-part-tool configured for bipolar electro-cauterization.

The at least one movable tool part of the multi-part-tool may be movable by a tension force provided by the rod, particularly movable by each of a tension force and a compression force provided by the rod.

The at least one movable part of the at least one multi-part-tool configured for bipolar electric cauterization may be electrically insulated from another part of said multi-part-tool.

The system, particularly the data-processing system, may be configured for processing toolset information data relating to the tool set, particularly to the tool set in a configuration mounted to the toolset interface.

The toolset information data may relate to the tool set that the magazine comprises.

The toolset information data may comprise an indication of a type of each tool of the toolset.

The toolset information data may comprise an indication of a functionality of each tool of the toolset.

The toolset information data may comprise, for each tool of the tool set, an indication of a receiving location containing the respective tool. In other words, in embodiments where the magazine comprises the tool set, the toolset information data may comprise the indication of the receiving location containing a tool when the magazine is mounted to the toolset interface.

The system, particularly the data-processing system, may be configured for processing a toolset identifier.

The system, particularly the data-processing system, may be configured for determining the toolset identifier based on an identification element associated with the tool set.

The tool set may comprise the identification element.

The packaging may comprise the identification element.

The data-exchange component may be configured for reading the toolset identifier from the identification element.

The data-processing system may be configured for receiving data relating to the toolset information data from a server.

For example, the data-processing system may be configured sending the toolset identifier to the server and receiving the toolset information data from the server, or by receiving data listing toolset identifiers and corresponding toolset information data from the server and selecting the appropriate toolset information data thereof.

The toolset identifier may be representative of at least one of an identity of the toolset, such as a serial number or an inventory number, and a type of the toolset, such as types, locations and/or quantities of the tools of the toolset.

The identification element may be configured to resist sterilization, particularly low-temperature sterilization, such as H2O2-sterilization.

The system may be configured for generating the alignment data. The alignment data may be indicative of the receiving location aligned with the tool activation mechanism, particularly indicative of the receiving location aligned with the patient component.

For example, the alignment data may comprise an indication of the rotational position of the magazine relative to the patient component, an ordinal number or an indication of the receiving location aligned with patient component.

The system may be configured for generating the alignment data at least by sensing the position and/or the orientation of the magazine with respect to the tool activation system, particularly with respect to the patient component.

The position and/or the orientation of the magazine with respect to the tool activation system, particularly with respect to the patient component, may be the rotational position of the magazine with respect to the tool activation mechanism, particularly with respect to the patient component.

The system may be configured for generating the alignment data at least by sensing the rotational position and/or the orientation of the motor system.

For example, the tool interface may be configured for receiving the magazine only with the predetermined angular offset vis-à-vis the coupling of the motor system. In other words, for example, at a given position/orientation of the output shaft of the motor system, only one orientation of the magazine may be possible.

The system, particularly the data-processing system, may be configured for processing receiving-location-data for at least one of the activated tool of the tool set and the receiving location aligned with the tool activation mechanism.

The magazine may comprise the at least one of the plurality of receiving location elements. Each receiving location element may be associated with a receiving location. In other words, at least one of the receiving locations may comprise a receiving location element.

The magazine may comprise the plurality of receiving location elements. Each receiving location may comprise a receiving location element.

The system, particularly the data-exchange component and/or a receiving location sensor, may be configured for sensing the receiving location element associated with the receiving location aligned with the patient component. Additionally or alternatively, the system, particularly the data-exchange component and/or a receiving location sensor, may be configured for sensing the presence of a receiving location element associated with the receiving location aligned with the patient component.

The system, particularly the data-processing system, may be configured for generating the receiving-location-data based on the sensed receiving location element.

The at least one or the plurality of receiving location elements may be selected from
- a plurality of magnets,
- a portion of the magazine comprising a protrusion or indentation,
- a marking on the magazine configured to be read by an optical rotation sensor, and/or
- an RFID-element.

The system, particularly the receiving location sensor, may comprise
- at least one sensor selected from a hall sensor and/or a reed switch,
- a mechanical switch, which mechanical switch is configured for engaging with the portion of the magazine comprising the protrusion or indentation,
- an optical rotation sensor associated configured for reading an optical marking on the magazine, and/or
- an RFID-reader configured to receive data from an RFID-element.

For example, the remainder of the surgery device may comprise the receiving location sensor.

The system, particularly the surgery device, may comprise the switching component configured for inhibiting provision of power to the tool based on the sensed receiving location element.

As set out above, the switching component may for example comprise the mechanical switch or the relay configured to inhibit provision of power or power for monopolar electro-cauterization.

The receiving-location-data may indicate at least whether a tool is configured
(a) for monopolar electro-cauterization or
(b) for at least one of bipolar electro-cauterization and/or no electro-cauterization.

The receiving-location-data may also indicate at least whether a tool is configured
(a) for monopolar electro-cauterization,
(b) for bipolar electro-cauterization, or
(c) not configured for electro-cauterization.

The receiving-location-data may also indicate at least whether a tool is
(a) configured for electro-cauterization, or
(b) not configured for electro-cauterization.

The system, particularly the data-processing system, may be configured for processing tool type data. The tool type data may relate to the at least one tool mounted to the distal portion of the patient component.

Each tool may comprise a tool data element. The system, particularly the data-processing system, may be configured for generating the tool type data based on a tool data element, e.g., a tool data element of the respective tool.

Each tool data element may be selected from at least one of a geometrical element of the tool, a magnetic portion of the tool, an optical marking of the tool, an RFID-element of the tool and a resistance of the tool.

The tool data elements may be of the same type. In other words, the tools may comprise tool data elements of the same type, such as each tool comprising an RFID-element, each tool comprising a magnetic portion, or each tool comprising an optical marking and an RFID-element.

Generating the tool type data may comprise sensing a resistance of the activated tool, that is, of the tool in the mounted state.

Processing the tool type data may comprise determining the tool type data relating to the at least one tool mounted to the distal portion of the patient component based on the toolset information data and the alignment data.

The system may be configured for the outputting device data. The device data may comprise the indication of the tool type data.

The device data may further comprise the indication of the receiving-location-data.

The device data may further comprise the indication of the status of the surgery device.

Outputting the device data, particularly the indication of the tool type data, may comprises outputting the indication of the tool type data together with the image data generated by the camera by means of the camera screen.

Outputting the indication of the tool type data may comprise outputting the indication of the tool type data by means of the additional screen connected to the data-processing system.

The surgery device may comprise an optical output element, such as an LCD-screen or a plurality of LEDs mounted to the surgery device.

The system, e.g., the data-processing system or an analogue circuit of the system, may be configured for electrically controlling the optical output element to output the indication of the tool type data. The optical output element may be a digital output element, such as an output element receiving a digital input.

The surgery device may comprise the mechanical output element, such as the plurality of moveable optical markers. The mechanical output element may be mechanically coupled to the magazine in the mounted configuration of the magazine.

The mechanical output element may be configured for outputting the indication of the tool type data. That is, optionally, the coupling of the mechanical output element and the magazine may provide for a change of an output of the mechanical output element when the receiving location aligned with the patient component changes. For example, a different marker may be visible on an outside of the device.

The system may comprise a connection to the acoustic output device, such as the speaker and/or the earphones. The system may be configured for outputting the acoustic indication of the tool type data.

The system may be configured for providing the power for electro-cauterization to the at least one first tool of the tool set by means of the power supply. The system may further be configured for not providing power for electro-cauterization to the at least one second tool of the tool set.

The system may be configured for providing power for monopolar electro-cauterization to at least one tool of the tool set, particularly by means of the power supply, and for not providing power for monopolar electro-cauterization to still at least one other tool of the tool set.

For example, the system may be configured for being operated in at least two surgery modes, the operation modes comprising the mode for monopolar electro-cauterization.

The system may be configured for activating said at least two surgery modes.

The system may be configured for providing power for monopolar electro-cauterization to at least one tool of the tool set, power for bipolar electro-cauterization to at least one other tool of the tool set, particularly by means of the power supply, and not providing power for electro-cauterization to still at least one other tool of the tool set.

The surgery modes may comprise a surgery mode for monopolar electro-cauterization, a surgery mode for bipolar electro-cauterization, and a surgery mode without cauterization.

The system may be configured for activating said surgery modes.

The device data may comprise an indication of a current surgery mode to be activated, such as a surgery mode corresponding to the activated tool

The system, particularly the data-processing system, may be configured for generating the electro-cauterization instruction data. The system may be configured for providing power for electro-cauterization to the at least one first tool based on the electro-cauterization instruction data by means of the power supply.

The system, particularly the data-processing system, may be configured for generating the electro-cauterization instruction data. The system may be configured for activating a surgery mode based on the electro-cauterization instruction data.

The system may comprise one or more relays, electronic switching units, such as power transistors, a toggle switch or the like to provide a connection of the suitable portion of the power supply and the activated tool and/or a connection of the suitable portion of the power supply and the activation element. Thus, the system may be configured for activating the surgery modes. For example, the data-processing system may be configured for controlling said relay(s), electronic switching unit(s), the toggle switch or the like.

The system, particularly the data-processing system, may be configured for generating the electro-cauterization instruction data at least based on the toolset information data and the alignment data. For example, the system may be configured for generating the electro-cauterization instruction data based on the equipment of the magazine/tool set and the position of magazine/selected element of toolset

The system, particularly the data-processing system, may be configured for generating the electro-cauterization instruction data at least based on the receiving-location-data.

For example, the system, particularly the data-processing system, may be configured for generating the electro-cauterization instruction data based on the receiving location element.

The system, particularly the data-processing system, may be configured for generating the electro-cauterization instruction data at least based on the alignment data.

The magazine may comprise the first set of receiving locations of the magazine only comprising one or more tools for monopolar electro-cauterization, and the second set of receiving locations of the magazine only comprising one or more tools for bipolar electro-cauterization and/or for no electro-cauterization.

Each set of receiving locations of the magazine may be arranged at pre-determined regions of the magazine. The regions may be discontinuous.

In some embodiments, the first set of receiving locations of the magazine may optionally only comprise one or more tools for monopolar electro-cauterization, and the second set of receiving locations of the magazine may optionally only comprise one or more tools for bipolar electro-cauterization, and the third set of receiving locations may optionally only comprises one or more tools not intended for electro-cauterization.

The system, particularly the data-processing system, may be configured for generating the electro-cauterization instruction data at least based on the tool type data generated based on the tool data element of the activated tool.

The system may be configured for activating the surgery mode for monopolar electro-cauterization based on the toolset information data and the alignment data, particularly based on the tool type data generated based on the toolset information data and the alignment data, and further only if the receiving-location-data corresponding to an activated tool indicate that the mounted tool is configured for monopolar electro-cauterization.

The system may be configured for activating the surgery modes for bipolar electro-cauterization and no electro-cauterization based on the toolset information data and the alignment data, particularly based on the tool type data generated based on the toolset information data and the alignment data.

The system being configured for only activating the surgery mode for monopolar electro-cauterization if the receiving-location-data corresponding to the activated tool indicate that the mounted tool is configured for monopolar electro-cauterization may comprise the switching component being configured for inhibiting activation of the surgery mode for monopolar electro-cauterization and/or inhibiting power transmission from the power supply to the at least one tool.

As discussed with respect to the method, for example, the switching component may be configured for interrupting the connection of the suitable portion of the power supply and the activated tool and/or the suitable portion of the power supply and the activation element when inhibiting the provision of power.

In other words, the switching component may be configured for inhibiting activation of the mode for monopolar electro-cauterization and/or configured for inhibiting power transmission from the power supply to the at least one tool based on the receiving-location-data, even if the alignment data and the toolset information data indicate that the mounted tool is configured for monopolar electro-cauterization.

The system, particularly the data-processing system, may be configured for only activating the surgery mode for monopolar electro-cauterization if a tool from the first set of receiving locations is activated. The system, particularly the data-processing system, may be configured for activating the surgery modes for bipolar electro-cauterization and no electro-cauterization based on the toolset information data and the alignment data, particularly based on the tool type data generated based on the toolset information data and the alignment data.

The system, particularly the power supply, may be configured for determining, upon the begin of bipolar electro-cauterization, whether the tool mounted to the distal portion of the patient component is currently suitable for bipolar electro-cauterization based on at least one electrical property of said tool, such as a resistance and/or an impedance of the tool mounted to the distal portion of the patient component.

For example, in case of a tool for monopolar electro-cauterization, a tool that is not intended for electro-cauterization, and/or a short-circuited tool for bipolar electro-cauterization, it may be determined that the respective tool is currently unsuitable for bipolar electro-cauterization based on the respective tool's resistance and/or the impedance.

The system may be configured for preventing the movement of the receiving locations, particularly of the magazine, with respect to the tool activation mechanism while a tool is mounted to the distal portion of the patient component.

As disclosed in the above-mentioned application EP24151941.2, this may, e.g., be achieved by appropriate control of the motor system, by use of pins and/or a lockable transmission of the motor system, and/or by a suitable mechanical design of the tool activation system.

The system, particularly the data-processing system, may be configured for storing the settings for electro-cauterization for at least one of the tools for monopolar and/or at least one of the tools for bipolar electro-cauterization.

For example, the settings may comprise a specific high-frequency electro-cauterization mode, a high-frequency intensity, a high-frequency duty cycle and/or a high-frequency waveform may be stored.

At least one of the settings may relate to a single tool.

At least one of the settings may relates to all tools for monopolar/bipolar electro-cauterization.

The system, particularly the data-processing system, may be configured for receiving the settings for electro-cauterization by the user input, such as the voice input and/or the button interface of the surgery device.

The system may be configured for providing power to the tools by means of the power supply based on the stored settings.

The surgery device may be the microinvasive surgery device.

The system, particularly the data-processing system, may be configured for receiving at least one tool selection input from the user. The tool selection input may be indicative of a tool to be mounted.

The system, particularly the data-processing system, may be configured for receiving the at least one tool selection input by means of the voice input and/or the button interface of the surgery device.

The system may be configured for mounting the selected tool in response to receiving the tool selection input from the user.

The system, particularly the data-processing system, may be configured for generating the tool suggestion and outputting the tool suggestions.

Generating the tool suggestion may be based on tool use data relating to the use of tools and/or the use of the surgery device.

The system may be configured for carrying out the method according to any of the method embodiments.

The method may comprise using the above-disclosed system.

The following embodiments also form part of the invention.

### Method embodiments

Below, embodiments of a method will be discussed. The method embodiments are abbreviated by the letter "M" followed by a number. Whenever reference is herein made to the "method embodiments", these embodiments are meant.
M1. A method comprising using
   - a surgery device 100, comprising
      o a patient component 110, particularly configured to be inserted into a body of a patient,
      ∘ a tool activation system 120, and
      ∘ a toolset interface 140;
   - a data-processing system 200; and
   - a tool set 241 comprising a plurality of tools 240.
M2. The method according to the preceding embodiment, wherein the method comprising mounting the tool set 241 to the toolset interface 140.
M3. The method according to the preceding embodiment, wherein the method comprises using a magazine 242 comprising the tool set 241, wherein mounting the tool set 241 to the toolset interface 140 is mounting the magazine 242 to the toolset interface 140.
M4. The method according to any of the preceding embodiments, wherein the method comprises activating at least one of the tools 240; and
   wherein activating the at least one tool 240 comprises mounting the at least one tool 240 to a distal portion of the patient component 110 by the tool activation system 120.
M5. The method according to the preceding embodiment and with the features of M3,
   wherein the surgery device 100 comprises a motor system 122;
   wherein the magazine 242 comprises a plurality of receiving locations 244, such as a plurality of chambers; and
   wherein activating the at least one tool 240 comprises aligning one of the receiving locations 244 with the tool activation mechanism 120 by the motor system 122.
M6. The method according to the preceding embodiment, wherein the method comprises the motor system 122 rotating the magazine 242 about a rotation axis when mounted to the toolset interface 140.
M7. The method according to any of the preceding embodiments with the features of M5, wherein the patient component is a tube component; and wherein aligning the one of the receiving locations 244 with the tool activation mechanism by the motor system is aligning said receiving location 244 with the patient component 110, such as the tube component.
M8. The method according to any of the preceding embodiments, particularly with the features of M3, wherein mounting the tool set 241, particularly the magazine 242, to the to the toolset interface 140 comprises opening a packaging 250 of the tool set 241, particularly of the magazine 242.
M9. The method according to any of the preceding embodiments, wherein the method comprises processing toolset information data relating to the tool set 241, particularly to the tool set mounted to the toolset interface.
M10. The method according to the preceding embodiment and with the features of M3, wherein the toolset information data relates to the tool set 241 that the magazine 242 comprises.
M11. The method according to any of the preceding embodiments with the features of M9, wherein the toolset information data comprises an indication of a type of each tool of the toolset.
M12. The method according to any of the preceding embodiments with the features of M9, wherein the toolset information data comprises an indication of a functionality of each tool of the toolset.
M13. The method according to any of the preceding embodiments with the features of M5 and M10, wherein the toolset information data comprises, for each tool of the tool set, an indication of a receiving location containing the respective tool.
M14. The method according to any of the preceding embodiments with the features of M9, wherein processing the toolset information data comprises processing a toolset identifier.
M15. The method according to the preceding embodiment,
   wherein the method comprises determining the toolset identifier based on an identification element 252 associated with the tool set 241.
M16. The method according to the preceding embodiment and with the features of at least one of M3 and M8, wherein at least one of the packaging 250 and the magazine 242 comprises the identification element 252.
M17. The method according to any of the preceding embodiments, wherein the data-processing system comprises a data-exchange component 212; and
   wherein determining the toolset identifier based on the identification element 252 comprises reading the toolset identifier from the identification element 252 by means of the data-exchange component 212.
M18. The method according to any of the preceding embodiments with the features of M14, wherein processing the toolset identifier comprises the data-processing system receiving data relating to the toolset information data from a server 300, such as sending the toolset identifier to the server 300 and receiving the toolset information data from the server 300, or by receiving data listing toolset identifiers and corresponding toolset information data.
M19. The method according to any of the preceding embodiments with the features of M5, wherein the method comprises generating alignment data, wherein the alignment data are indicative of the receiving location 244 aligned with the tool activation mechanism, particularly the receiving location 244 aligned with the patient component 110.
M20. The method according to any of the preceding embodiments with the features of M19, wherein generating the alignment data comprises determining a position and/or an orientation of the magazine with respect to the tool activation system 120, particularly with respect to the patient component 110.
M21. The method according to any of the preceding embodiments with the features of M19, wherein generating the alignment data comprises sensing a rotational position of the magazine 242 with respect to the tool activation mechanism 120, particularly with respect to the patient component 110.
M22. The method according to any of the preceding embodiments with the features of M19, wherein generating the alignment data comprises sensing a rotational position and/or an orientation of the motor system 122, particularly wherein the tool interface is configured for receiving a magazine only with a predetermined angular offset vis-à-vis a coupling of the motor system 122.
M23. The method according to any of the two preceding embodiments, wherein sensing the rotational position of the magazine and/or the rotational position of the motor system comprises using at least one of
   - a rotary encoder,
   - a plurality of magnets and at least one sensor selected from a hall sensor and/or a reed switch,
   - a mechanical switch configured for engaging with a part of the magazine, such as an indentation or a protrusion of the magazine or a part of the motor system,
   - an optical rotation sensor, and/or
   - an RFID-element and a corresponding reader.
M24. The method according to any of the preceding embodiments with the features of M5, wherein the method comprises processing receiving-location-data for at least one of the activated tool of the tool set and the receiving location aligned with the tool activation mechanism.
M25. The method according to the preceding embodiment and with the features of M5, wherein processing the receiving-location-data comprises sensing, by a remainder of the surgery device 100, a receiving location element associated with the receiving location aligned with the patient component and/or a presence of a receiving location element associated with the receiving location aligned with the tool activation mechanism.
M26. The method according to the preceding embodiment, wherein the method comprises generating the receiving-location-data based on the sensed receiving location element.
M27. The method according to any of the preceding embodiments with the features of M25, wherein the receiving location element and a corresponding sensor associated with a remainder of the surgery device are selected from
   - a plurality of magnets and at least one sensor selected from a hall sensor and/or a reed switch associated with the remainder of the surgery device,
   - a portion of the magazine comprising a protrusion or indentation of the magazine and a mechanical switch associated with the remainder of the surgery device, which mechanical switch is configured for engaging with said portion of the magazine,
   - a marking on the magazine configured to be read by an optical rotation sensor associated with the remainder of the surgery device, and/or
   - an RFID-element and a corresponding reader associated with a remainder of the surgery device.
M28. The method according to any of the preceding embodiments with the features of M25, wherein the sensor comprises a switching component, wherein the switching component inhibits provision of power to the tool based on the sensed receiving location element.
M29. The method according to any the preceding embodiments with the features of M24, wherein the receiving-location-data indicate at least whether a tool is configured
   (a) for monopolar electro-cauterization, or
   (b) for at least one of bipolar electro-cauterization and/or no electro-cauterization.
M30. The method according to the preceding embodiment, wherein the receiving-location-data indicate at least whether a tool is configured
   (a) for monopolar electro-cauterization,
   (b) for bipolar electro-cauterization, or
   (c) not configured for electro-cauterization.
M31. The method according to any the preceding embodiments with the features of M24, wherein the receiving-location-data indicate at least whether a tool is
   (a) configured for electro-cauterization, or
   (b) not configured for electro-cauterization.
M32. The method according to any of the preceding embodiments with the features of M4, wherein the method comprises processing tool type data, wherein the tool type data relate to the at least one tool mounted to the distal portion of the patient component.
M33. The method according to the preceding embodiment, wherein the method comprises generating the tool type data based on a tool data element, and wherein each tool comprises a tool data element.
M34. The method according to the preceding embodiment, wherein each tool data element is selected from at least one of a geometrical element of the tool, a magnetic portion of the tool, an optical marking of the tool, an RFID-element of the tool and a resistance of the tool.
M35. The method according to the preceding embodiment, wherein the tool data elements are of a same type.
M36. The method according to any of the three preceding embodiments, wherein generating the tool type data comprises sensing a resistance of the activated tool.
M37. The method according to any of the preceding embodiments with the features of M32, M19 and M9, wherein processing the tool type data comprises determining the tool type data relating to the at least one tool mounted to the distal portion of the patient component based on the toolset information data and the alignment data.
M38. The method according to any of the preceding embodiments with the features of M32, wherein the method comprises outputting device data, particularly wherein the device data comprise an indication of the tool type data.
M39. The method according to any of the preceding embodiment, wherein the device data further comprise an indication of a status of the surgery device.
M40. The method according to any of the preceding embodiments with the features of M38, wherein the method comprises using a camera and a camera screen outputting image data captured by the camera, particularly wherein the image data relate to an inside of a body of the patient, and
   wherein outputting the device data, particularly the indication of the tool type data, comprises outputting the indication of the tool type data together with the image data generated by the camera by means of the camera screen 210.
M41. The method according to any of the preceding embodiments with the features of M38, wherein the method comprises using a camera and a camera screen outputting image data captured by the camera, particularly wherein the image data relate to an inside of a body of the patient, and
   wherein outputting the indication of the tool type data comprises outputting the indication of the tool type data by means of an additional screen connected to the data-processing system 200.
M42. The method according to any of the preceding embodiments with the features of M38, wherein the surgery device comprises an optical output element, such as an LCD-screen or a plurality of LEDs mounted to the surgery device, and
   wherein the method comprises electrically controlling the optical output element to output an indication of the tool type data, particularly wherein the optical output element is a digital output element.
M43. The method according to any of the preceding embodiments with the features of M38, particularly with the features of M5, wherein the surgery device comprises a mechanical output element, such as a plurality of moveable optical markers, wherein the mechanical output element is mechanically coupled to the magazine in a mounted configuration, and
   wherein the method comprises outputting an indication of the tool type data by means of the mechanical output element.
M44. The method according to any of the preceding embodiments with the features of M38, wherein the method comprises outputting an acoustic indication of the tool type data, e.g. by means of a speaker.
M45. The method according to any of the preceding embodiments, wherein the method comprises providing power for electro-cauterization to at least one first tool 240 of the tool set 241 by means of a power supply 220, and wherein the method comprises not providing power for electro-cauterization to at least one second tool 240 of the tool set 241.
M46. The method according to the preceding embodiment, wherein the method comprises providing power for monopolar electro-cauterization to at least one tool of the tool set, particularly by means of the power supply 220, and not providing power for monopolar electro-cauterization to still at least one other tool of the tool set, and
   wherein particularly, the method comprises at least two surgery modes, the surgery modes comprising a surgery mode for monopolar electro-cauterization using the power supply 220.
M47. The method according to any of the two preceding embodiments, wherein the method comprises providing power for monopolar electro-cauterization to at least one tool of the tool set, power for bipolar electro-cauterization to at least one other tool of the tool set, particularly by means of the power supply 220, and not providing power for electro-cauterization to still at least one other tool of the tool set, and
   wherein the surgery modes comprise
   - a surgery mode for monopolar electro-cauterization using the power supply 220,
   - a surgery mode for bipolar electro-cauterization using the power supply, and
   - a surgery mode without cauterization.
M48. The method according to any of the preceding embodiments with the features of M38, wherein the device data comprise an indication of a current surgery mode to be activated, such as a surgery mode corresponding to the activated tool.
M49. The method according to any of the preceding embodiments with the features of M45, wherein the method comprises generating electro-cauterization instruction data, wherein the method comprises the power supply 220 providing power for electro-cauterization to the at least one first tool 240 based on the electro-cauterization instruction data.
M50. The method according to any of the preceding embodiments with the features of M49 and at least one of M46 and M47, wherein the method comprises generating the electro-cauterization instruction data, wherein the method comprises providing power for electro-cauterization according to a surgery mode activated based on the electro-cauterization instruction data.
M51. The method according to the two preceding embodiments and with the features of M9 and M19, particularly with the features of M37, wherein the method comprises generating the electro-cauterization instruction data at least based on the toolset information data and the alignment data.
M52. The method according to any of the preceding embodiments with the features of M24 and M49, particularly with the features of M25, wherein the method comprises generating the electro-cauterization instruction data at least based on the receiving-location-data.
M53. The method according to any of the preceding embodiments with the features of M5, M19, M22 and M49, wherein the method comprises generating the electro-cauterization instruction data at least based on the alignment data, and
   wherein the magazine comprises
      - a first set of receiving locations of the magazine only comprising one or more tools for monopolar electro-cauterization, and
      - a second set of receiving locations of the magazine only comprising one or more tools for bipolar electro-cauterization and/or for no electro-cauterization;
   wherein each set of receiving locations of the magazine is arranged at pre-determined regions of the magazine.
M54. The method according to the preceding embodiment, wherein the first set of receiving locations of the magazine only comprises one or more tools for monopolar electro-cauterization, and the second set of receiving locations of the magazine only comprises one or more tools for bipolar electro-cauterization, and a third set of receiving locations only comprises one or more tools not intended for electro-cauterization.
M55. The method according to any of the preceding embodiments with the features of M33 and M49, wherein the method comprises generating the electro-cauterization instruction data at least based on the tool type data generated based on the tool data element of the activated tool.
M56. The method according to any of the preceding embodiments with the features of M29, M50, and M51, wherein the surgery mode for monopolar electro-cauterization is activated based on the toolset information data and the alignment data, particularly based on the tool type data generated based on the toolset information data and the alignment data, and further only if the receiving-location-data corresponding to an activated tool indicate that the mounted tool is configured for monopolar electro-cauterization.
M57. The method according to the preceding embodiment, wherein the surgery modes for bipolar electro-cauterization and no electro-cauterization are activated based on the toolset information data and the alignment data, particularly based on the tool type data generated based on the toolset information data and the alignment data.
M58. The method according to any of the two preceding embodiments, wherein the step of only activating the surgery mode for monopolar electro-cauterization if the receiving-location-data corresponding to an activated tool indicate that the mounted tool is configured for monopolar electro-cauterization comprises the switching component inhibiting activation of the surgery mode for monopolar electro-cauterization and/or inhibiting power transmission from the power supply to the at least one tool.
M59. The method according to any of the preceding embodiments with the features of M29, M51 and M53, wherein the surgery mode for monopolar electro-cauterization is only activated if a tool from the first set of receiving locations is activated, and
   wherein the surgery modes for bipolar electro-cauterization and no electro-cauterization are activated based on the toolset information data and the alignment data, particularly based on the tool type data generated based on the toolset information data and the alignment data.
M60. The method according to any of the preceding embodiments with the features M46, wherein the method comprises, upon a begin of bipolar electro-cauterization, determining whether the tool mounted to the distal portion of the patient component is currently suitable for bipolar electro-cauterization based on at least one electrical property of said tool, such as a resistance and/or an impedance of the tool mounted to the distal portion of the patient component.
M61. The method according to any of the preceding embodiments with the features of M5, wherein the method comprises preventing a movement of the receiving locations, particularly of the magazine, with respect to the tool activation mechanism while a tool is mounted to the distal portion of the patient component.
M62. The method according to any of the preceding embodiments with the features of M34 and M45, particularly with the features of M14, wherein the method comprises storing settings for electro-cauterization for at least one of the tools for monopolar and/or at least one of the tools for bipolar electro-cauterization.
M63. The method according to the preceding embodiment, wherein at least one of the settings relates to a single tool.
M64. The method according to any of the two preceding embodiments, wherein at least one of the settings relates to all tools for monopolar/bipolar electro-cauterization.
M65. The method according to any of the preceding embodiments with the features of M45, particularly with the features of M62, wherein the method comprises receiving the settings for electro-cauterization by a user input, such as a voice input and/or a button interface of the surgery device.
M66. The method according to any of the preceding embodiments with the features of M62, wherein the method comprises the power supply providing power to the tools based on the stored settings.
M67. The method according to any of the preceding embodiments, wherein the surgery device is a microinvasive surgery device.
M68. The method according to any of the preceding embodiments with the features of M4, wherein the method comprises receiving at least one tool selection input from a user, the tool selection input being indicative of a tool to be mounted.
M69. The method according to the preceding embodiment, wherein the method comprises receiving the at least one tool selection input by means of a voice input, a button interface of the surgery device and/or a control interface for a robotic surgery system.
M70. The method according to any of the preceding embodiments with the features of M68, wherein the method comprises, in response to receiving a tool selection input, mounting a selected tool.
M71. The method according to any of the preceding embodiments, wherein the method comprises generating a tool suggestion and outputting the tool suggestions.
M72. The method according to the preceding embodiment, wherein generating the tool suggestion is based on tool use data relating to a use of tools and/or the surgery device.
M73. The method according to any of the preceding embodiments, wherein the surgery device is a robotic surgery device, particularly a robotic microinvasive surgery device.
M74. The method according to any of the preceding embodiments, wherein the operating element is robotically actuated.
M75. The method according to any of the preceding embodiments, wherein the surgery device is a manually operated device and/or a handheld device, particularly a manually operated microinvasive surgery device and/or a handheld microinvasive surgery device.

### Surgery device embodiments

Below, embodiments of a surgery device will be discussed. The surgery device embodiments are abbreviated by the letter "SD" followed by a number. Whenever reference is herein made to the "surgery device embodiments", these embodiments are meant.
SD1. A surgery device comprising
   - a body element 114;
   - a patient component 110 comprising a distal portion;
   - a toolset interface 140 configured for receiving a tool set 241 comprising a plurality of tools 240;
   - a tool activation system 120, wherein the tool activation system 120 is configured for mounting at least one tool 240 of the tool set to the distal portion of the patient component 110; and
   - an operating element;
   particularly wherein the distal portion of the patient component is configured to be introduced into a body of a patient.
SD2. The surgery device according to the preceding embodiment, wherein the patient component 110 is a tube component comprising a proximal portion and a distal portion, wherein the proximal portion of the tube component 110 is connected to the body element 114.
SD3. The surgery device according to any of the preceding surgery device embodiments, wherein the toolset interface 140 is configured for receiving a magazine 242 comprising the tool set 241, particularly configured for receiving the plurality of tools of the set at a same time.
SD4. The surgery device according to any of the preceding surgery device embodiments, wherein the tool activation system 120 is configured for activating at least one tool 240 of the tool set 241 by mounting the at least one tool 240 to the distal portion of the patient component.
SD5. The surgery device according to the preceding two embodiments, wherein the tool activation system 120 is configured for moving the at least one tool 240 from the magazine 242, particularly for mounting the at least one tool to the distal portion of the patient component.
SD6. The surgery device according to the preceding embodiment, wherein the tool activation system 120 is configured for deactivating the at least one tool 240 by unmounting the at least one tool 240 from the distal portion of the patient component 110, particularly wherein unmounting the at least one tool 240 comprises moving the at least one tool 240 to the magazine 242.
SD7. The surgery device according to any of the preceding surgery device embodiments, wherein the tool activation system 120 is configured for interchanging the tool 240 mounted to the distal portion of the patient component 110, particularly while the distal portion of the patient component 110 is in a surgery configuration.
SD8. The surgery device according to any of the preceding surgery device embodiments, wherein the tool activation system 120 comprises a tool coupling element 116 configured for releasably coupling one of the tools 240 to the tool activation system, particularly for releasably coupling one tool at a time to the tool activation system.
SD9. The surgery device according to the preceding embodiment, wherein the tool coupling element 116 is configured for being coupled to the tool 240 and for being decoupled from the tool 240 in a magazine configuration.
SD10. The surgery device according to the preceding embodiment and with the features of SD2, wherein the tool coupling element 116 is configured for being moved along an inside of the tube component from the magazine 242 configuration towards the distal portion of the tube component, and
   wherein the tool coupling element 116 is in a mounted configuration when the tool 240 is mounted to the distal portion of the tube component.
SD11. The surgery device according to any of the preceding surgery device embodiments, wherein the body element 114 comprises the toolset interface 140.
SD12. The surgery device according to any of the preceding surgery device embodiments, particularly with the features of SD3,
   wherein the surgery device comprises a motor system 122, and wherein the motor system 122 is configured for moving the tool set 241 coupled to the toolset interface 140 with respect to the body element 114, particularly for moving the magazine 242 coupled to the body element 114 with respect to the body element 114.
SD13. The surgery device according to the preceding embodiment, wherein the motor system 122 is configured for rotating the magazine 242 about a rotation axis when mounted to the toolset interface 140.
SD14. The surgery device according to any of the preceding surgery device embodiments with the features of SD3 and SD12, wherein the toolset interface 140 is configured for receiving a magazine 242 comprising a plurality of tool receiving locations 244, wherein each tool receiving location 244 is configured for receiving at least one tool, and
   wherein the motor system 122 is configured for moving the magazine 242 in the toolset interface 140 so as to align one tool receiving location 244 at a time with the patient component 110.
SD15. The surgery device according to any of the preceding surgery device embodiments with the features of SD14, wherein the surgery device is configured for sensing a position and/or an orientation of the magazine with respect to the tool activation system 120, particularly with respect to the patient component 110.
SD16. The surgery device according to the preceding embodiment, wherein the surgery device is configured for sensing a rotational position of the magazine 242 with respect to the tool activation mechanism 120, particularly with respect to the patient component 110.
SD17. The surgery device according to any of the preceding surgery device embodiments with the features of SD12 and SD14, wherein the surgery device is configured for sensing a rotational position and/or an orientation of the motor system 122, particularly wherein the tool interface is configured for receiving a magazine only with a predetermined angular offset vis-à-vis a coupling of the motor system 122.
SD18. The surgery device according to any of the preceding surgery device embodiments with the features of SD15, wherein the surgery device 100 comprises at least one magazine sensor selected from
   - a hall sensor and/or a reed switch,
   - a mechanical switch,
   - an optical rotation sensor, and/or
   - an RFID-reader,
   wherein the magazine sensor is configured for sensing the rotational position of the magazine 242 with respect to the tool activation mechanism 120, particularly with respect to the patient component 110.
SD19. The surgery device according to any of the preceding surgery device embodiments with the features of SD17, wherein the surgery device 100 comprises at least one motor sensor selected from
   - a rotary encoder,
   - a hall sensor and/or a reed switch,
   - a mechanical switch,
   - an optical rotation sensor, and/or
   - an RFID-reader,
   wherein the motor sensor is configured for sensing the rotational position of the motor system 122.
SD20. The surgery device according to any of the preceding surgery device embodiments with the features of SD4, wherein the surgery device is configured for preventing a movement of the receiving locations, particularly of the magazine, with respect to the tool activation mechanism while a tool is mounted to the distal portion of the patient component.
SD21. The surgery device according to any of the preceding surgery device embodiments, wherein the operating element is an operating lever.
SD22. The surgery device according to any of the preceding surgery device embodiments, wherein the tool activation system 120 comprises a rack 112 and/or a rod.
SD23. The surgery device according to any of the preceding surgery device embodiments with the features of SD8 and SD22, wherein the rack 112 and/or the rod are configured for transferring tension and compression to the mounted tool by means of the tool coupling element 116.
SD24. The surgery device according to any of the preceding surgery device embodiments with the features of SD2 and SD22, wherein the tool activation system 116 is configured for moving the at least one tool 240 from the magazine 242 to the distal portion of the tube component by moving the tool 240 along the inside of the tube component using the rack 112.
SD25. The surgery device according to the preceding embodiment, wherein the tool activation system 120 is further configured for moving the at least one tool from the distal portion of the tube component to the magazine 242 by moving the tool along the tube inside of the tube component using the rack 112.
SD26. The surgery device according to any of the preceding surgery device embodiments with the features of SD22 and SD2, wherein a distal end of the rack 112 is connected to the tool coupling element 116, and wherein the tool activation system 120 is configured for moving the tool coupling element 116 from a magazine configuration to a mounted configuration by means of the rack 112.
SD27. The surgery device according to any of the preceding surgery device embodiments with the features of SD2, wherein the tube component is configured for transmitting electric power for electro-cauterization to at least one mounted tool.
SD28. The surgery device according to any of the preceding surgery device embodiments with the features of SD27, wherein the body element 114 comprises a bore, such as a sleeve bearing, configured for receiving the tube component, and wherein the bore is made from a conductive material, such as stainless steel, and wherein the bore is configured for transmitting electric power from the body element 114 to the tube component.
SD29. The surgery device according to any of the preceding surgery device embodiments with the features of SD27, wherein the tube component comprises an electrically insulating outer layer and an electrically conductive layer within the insulating outer layer.
SD30. The surgery device according to any of the preceding surgery device embodiments with the features of SD27, wherein the tube component comprises a cable configured for conducting electric power from the body element 114 to the at least one mounted tool 240.
SD31. The surgery device according to any of the preceding surgery device embodiments with the features of SD22, wherein at least a section of the rod is configured for transmitting electric power to the at least one tool 240.
SD32. The surgery device according to any of the preceding surgery device embodiments with the features of SD12 and SD22, particularly with the features of SD8, wherein the motor system 122 is configured for moving the tool coupling element 116 between the magazine configuration and the mounted configuration.
SD33. The surgery device according to the preceding embodiment, wherein the motor system 122 comprises a rack drive, wherein the rack drive comprises a gear engaging with the rack 112.
SD34. The surgery device according to the preceding embodiment, wherein the rack drive comprises a rack-drive-motor and a rack-drive-transmission, wherein the rack-drive-motor is connected to the rack-drive-transmission, and wherein the rack-drive-transmission is self-locking, particularly not backdrivable.
SD35. The surgery device according to the preceding embodiment, wherein the rack-drive-transmission comprises a self-locking worm-gear unit.
SD36. The surgery device according to the penultimate embodiment, wherein the rack-drive-transmission comprises an automatic locking mechanism.
SD37. The surgery device according to any of the preceding surgery device embodiments with the features of S4, wherein the microinvasive surgery device, particularly the motor system 122, comprises a magazine drive configured for moving the magazine so as to assume a position of the plurality of positions corresponding to the receiving locations.
SD38. The surgery device according to any of the preceding surgery device embodiments with the features of SD12, wherein the motor system 122 comprises a gear stage, such as a planetary gear, configured to enable moving the tool set 241 coupled to the toolset interface 140 with respect to the body element 114.
SD39. The surgery device according to any of the preceding surgery device embodiments, wherein the surgery device comprises an input interface, such as a button interface.
SD40. The surgery device according to any of the preceding surgery device embodiments, wherein the surgery device is a microinvasive surgery device.
SD41. The surgery device according to any of the preceding surgery device embodiments, wherein the surgery device is a robotic surgery device, particularly a robotic microinvasive surgery device.
SD42. The surgery device according to any of the preceding surgery device embodiments, wherein the operating element is robotically actuated.
SD43. The surgery device according to any of the preceding surgery device embodiments, particularly with the features of SD21, wherein the surgery device is a manually operated device and/or a handheld device, particularly a manually operated microinvasive surgery device and/or a handheld microinvasive surgery device.
SD44. The surgery device according to any of the preceding surgery device embodiments, wherein the surgery device is configured for performing the method according to any of the method embodiments.
M76. The method according to any of the preceding method embodiments, wherein the method comprises using a surgery device according to any of the surgery device embodiments.

### System embodiments

Below, embodiments of a system will be discussed. The system embodiments are abbreviated by the letter "S" followed by a number. Whenever reference is herein made to the "system embodiments", these embodiments are meant.
S1. A system, comprising
   - a surgery device 100 according to any of the surgery device embodiments;
   - a tool set 241 comprising a plurality of tools 240;
   - a power supply 220 configured for providing electric power for electric cauterization; and
   - a data-processing system 200.
S2. The system according to the preceding embodiment, wherein the system further comprises a user-interface device.
S3. The system according to any of the preceding embodiments with the features of S2, wherein the user-interface device comprises a camera screen 210 configured for outputting image data generated by the camera for microinvasive surgery.
S4. The system according to the preceding embodiment, wherein the user-interface device further comprises an additional screen 210, wherein the additional screen is connected to the data-processing system 200.
S5. The system according to any of the two preceding embodiments, wherein the system comprises the camera for microinvasive surgery.
S6. The system according to any of the preceding system embodiments, wherein the data-processing system 200 comprises a data-exchange component 212.
S7. The system according to any of the preceding system embodiments, wherein the system comprises a magazine 242 comprising the tool set 241, particularly wherein the surgery device is according to SD3 or any surgery device embodiment dependent thereon.
S8. The system according to any of the preceding system embodiments, particularly with the features of S7, wherein the tool set 241, particularly the magazine 242, is configured for being mounted to the toolset interface 140 of the surgery device 100.
S9. The system according to the any of the preceding embodiments with the features of S7, wherein the magazine 242 comprises a plurality of receiving locations 244, such as a plurality of chambers;
   wherein particularly, the surgery device comprises the features of SD12,
   wherein activating the at least one tool 240 comprises aligning one of the receiving locations 244 with the tool activation mechanism 120, particularly with the patient component 110; and
   wherein the motor system 122 is configured for aligning the one of the receiving locations 244 with the tool activation mechanism 120, particularly with the patient component 110.
S10. The system according to any of the preceding system embodiments, wherein the tool set, particularly the magazine, comprises a packaging.
S11. The system according to the preceding embodiment, wherein the packaging is a sterile packaging.
S12. The system according to any of the preceding embodiments with the features of S7, wherein the magazine is configured to be mounted to the toolset interface of the surgery device.
S13. The system to any of the preceding system embodiments, wherein at least one tool of the tool set 241 is a multi-part-tool, wherein the at least one multi-part-tool comprises at least one movable tool part, which at least one moveable tool part is configured for being moved relative to another part of the tool, such as a remainder of the tool or a second moveable part of the tool.
S14. The system according to the preceding embodiment, wherein the at least one multi-part-tool comprises a scissors tool and/or a forceps tool.
S15. The system according to any of the preceding embodiments with the features of S13, wherein the at least one movable tool part is movable by an application of force when the multi-part-tool is in a mounted configuration.
S16. The system according to any of the preceding system embodiments, wherein the tools 240 of the tool set 241 are tools for microinvasive surgery.
S17. The system according to any of the preceding system embodiments, wherein at least one tool 240 of the tool set 241 is a tool for electro-cauterization.
S18. The system according to any of the preceding embodiments with the features of S13, wherein the at least one multi-part-tool comprises at least one multi-part-tool configured for bipolar electro-cauterization.
S19. The system according to any of the preceding system embodiments with the features of S13, wherein the surgery device comprises the features of SD22, wherein the at least one movable tool part of the multi-part-tool is movable by a tension force provided by the rod, particularly movable by each of a tension force and a compression force provided by the rod.
S20. The system according to any of the preceding system embodiments with the features of S19, wherein the at least one movable part of the at least one multi-part-tool configured for bipolar electric cauterization is electrically insulated from another part of said multi-part-tool.
S21. The system according to any of the preceding system embodiments, wherein the system, particularly the data-processing system 200, is configured for processing toolset information data relating to the tool set 241, particularly to the tool set 241 in a configuration mounted to the toolset interface 140.
S22. The system according to any of the preceding system embodiments and with the features of S7, wherein the toolset information data relates to the tool set 241 that the magazine 242 comprises.
S23. The system according to any of the preceding system embodiments and with the features of S21, wherein the toolset information data comprises an indication of a type of each tool of the toolset.
S24. The system according to any of the preceding system embodiments and with the features of S21, wherein the toolset information data comprises an indication of a functionality of each tool of the toolset.
S25. The system according to any of the preceding system embodiments and with the features of S9 and S22, wherein the toolset information data comprises, for each tool of the tool set, an indication of a receiving location containing the respective tool.
S26. The system according to any of the preceding system embodiments with the features of S21, wherein the system, particularly the data-processing system 200, is configured for processing a toolset identifier.
S27. The system according to the preceding embodiment,
   wherein the system, particularly the data-processing system 200, is configured for determining the toolset identifier based on an identification element 252 associated with the tool set 241.
S28. The system according to the preceding embodiment, wherein the tool set 241 comprises the identification element 252.
S29. The system according to any of the two preceding system embodiments with the features of S10 and S26, wherein the packaging comprises the identification element 252.
S30. The system according to any of the preceding system embodiments with the features of S6 and S27, wherein the data-exchange component 212 is configured for reading the toolset identifier from the identification element 252.
S31. The system according to any of the preceding system embodiments with the features of S26, wherein the data-processing system 200 is configured for receiving data relating to the toolset information data from a server 300.
S32. The system according to any of the preceding system embodiments with the features of S26, wherein the toolset identifier is representative of at least one of an identity of the toolset, such as a serial number or an inventory number, and a type of the toolset, such as types, locations and/or quantities of the tools of the toolset.
S33. The system according to any of the preceding embodiments with the features of S27, wherein the identification element is configured to resist sterilization.
S34. The system according to any of the preceding system embodiments, wherein the system is configured for generating alignment data, wherein the alignment data are indicative of the receiving location 244 aligned with the tool activation mechanism, particularly the receiving location 244 aligned with the patient component 110;
   particularly wherein the surgery device 100 comprises the features of SD14.
S35. The system according to any of the preceding system embodiments with the features of S34, wherein the system is configured for generating the alignment data at least by sensing a position and/or an orientation of the magazine with respect to the tool activation system 120, particularly with respect to the patient component 110.
S36. The system according to the preceding embodiment, wherein the position and/or the orientation of the magazine with respect to the tool activation system 120, particularly with respect to the patient component 110, is a rotational position of the magazine 242 with respect to the tool activation mechanism 120, particularly with respect to the patient component 110.
S37. The system according to any of the preceding system embodiments with the features of S34, wherein the system is configured for generating the alignment data at least by sensing a rotational position and/or an orientation of the motor system 122,
   particularly wherein the tool interface is configured for receiving a magazine only with a predetermined angular offset vis-à-vis a coupling of the motor system 122.
S38. The system according to any of the preceding system embodiments with the features of S34, wherein the surgery device is corresponding to SD18 or any embodiments dependent thereon.
S39. The system according to any of the preceding embodiments with the features of S9, wherein the system, particularly the data-processing system 200, is configured for processing receiving-location-data for at least one of the activated tool of the tool set and the receiving location aligned with the tool activation mechanism.
S40. The system according to any the preceding system embodiments with the features of S9, wherein the magazine comprises at least one or a plurality of receiving location elements, wherein each receiving location element is associated with a receiving location.
S41. The system according to the preceding embodiment, wherein the magazine comprises the plurality of receiving location elements, wherein each receiving location comprises a receiving location element.
S42. The system according to any of the preceding two embodiments and with the features of S40, particularly with the features of S6, wherein the system, particularly the data-exchange component 212 and/or a receiving location sensor, is configured for sensing the receiving location element associated with the receiving location aligned with the patient component and/or a presence of a receiving location element associated with the receiving location aligned with the patient component.
S43. The system according to the preceding embodiment, wherein the system, particularly the data-processing system 200, is configured for generating the receiving-location-data based on the sensed receiving location element.
S44. The system according to any of the preceding embodiments with the features of S40, wherein the at least one or the plurality of receiving location elements is/are selected from
   - a plurality of magnets,
   - a portion of the magazine comprising a protrusion or indentation,
   - a marking on the magazine configured to be read by an optical rotation sensor, and/or
   - an RFID-element.
S45. The system according to any of the preceding embodiments with the features of S40, particularly with the features of S42 and S44, wherein the system, particularly the receiving location sensor, comprises
   - at least one sensor selected from a hall sensor and/or a reed switch,
   - a mechanical switch, which mechanical switch is configured for engaging with the portion of the magazine comprising the protrusion or indentation,
   - an optical rotation sensor associated configured for reading an optical marking on the magazine, and/or
   - an RFID-reader configured to receive data from an RFID-element.
S46. The system according to any of the preceding embodiments with the features of S42, wherein the system, particularly the surgery device, comprises a switching component configured for inhibiting provision of power to the tool based on the sensed receiving location element.
S47. The system according to any of the preceding system embodiments with the features of S39, wherein the receiving-location-data indicate at least whether a tool is configured
   (c) for monopolar electro-cauterization or
   (d) for at least one of bipolar electro-cauterization and/or no electro-cauterization.
S48. The system according to the preceding embodiment, wherein the receiving-location-data indicate at least whether a tool is configured
   (d) for monopolar electro-cauterization,
   (e) for bipolar electro-cauterization, or
   (f) not configured for electro-cauterization.
S49. The system according to any the preceding system embodiments with the features of S39, wherein the receiving-location-data indicate at least whether a tool is
   (c) configured for electro-cauterization, or
   (d) not configured for electro-cauterization.
S50. The system according to any of the preceding system embodiments with the features of S7, wherein the system, particularly the data-processing system 200, is configured for processing tool type data, wherein the tool type data relate to the at least one tool 240 mounted to the distal portion of the patient component 110.
S51. The system according to any of the preceding system embodiment with the features of S7, wherein each tool comprises a tool data element, and
   wherein the system, particularly the data-processing system, is configured for generating the tool type data based on a tool data element.
S52. The system according to the preceding embodiment, wherein each tool data element is selected from at least one of a geometrical element of the tool, a magnetic portion of the tool, an optical marking of the tool, an RFID-element of the tool and a resistance of the tool.
S53. The system according to the preceding embodiment, wherein the tool data elements are of a same type.
S54. The system according to any of the four preceding embodiments, wherein generating the tool type data comprises sensing a resistance of the activated tool.
S55. The system according to any of the preceding system embodiments with the features of S50, S34 and S21, wherein processing the tool type data comprises determining the tool type data relating to the at least one tool mounted to the distal portion of the patient component based on the toolset information data and the alignment data.
S56. The system according to any of the preceding system embodiments with the features of S50, wherein the system is configured for outputting device data, particularly wherein the device data comprise an indication of the tool type data.
S57. The system according to any of the two preceding embodiments, wherein the device data further comprise an indication of a status of the surgery device.
S58. The system according to any of the preceding system embodiments with the features of S3 and S56, wherein outputting the device data, particularly the indication of the tool type data, comprises outputting the indication of the tool type data together with the image data generated by the camera by means of the camera screen 210.
S59. The system according to any of the preceding system embodiments with the features of S4 and S56,
   wherein outputting the indication of the tool type data comprises outputting the indication of the tool type data by means of the additional screen connected to the data-processing system 200.
S60. The system according to any of the preceding system embodiments with the features of S56, wherein the surgery device comprises an optical output element, such as an LCD-screen or a plurality of LEDs mounted to the surgery device, and
   wherein the system is configured for electrically controlling the optical output element to output an indication of the tool type data, particularly wherein the optical output element is a digital output element.
S61. The system according to any of the preceding embodiments with the features of S56, particularly with the features of S9, wherein the surgery device comprises a mechanical output element, such as a plurality of moveable optical markers, wherein the mechanical output element is mechanically coupled to the magazine in a mounted configuration, and
   wherein the mechanical output element is configured for outputting the indication of the tool type data.
S62. The system according to any of the preceding system embodiments with the features of S56, wherein the system comprises a connection to an acoustic output device, such as a speaker and/or earphones, and wherein the system is configured for outputting an acoustic indication of the tool type data.
S63. The system according to any of the preceding system embodiments, wherein the system is configured for providing power for electro-cauterization to at least one first tool 240 of the tool set 241 by means of the power supply 220, and
   wherein the system is further configured for not providing power for electro-cauterization to at least one second tool 240 of the tool set 241.
S64. The system according to any of the preceding system embodiments, wherein the system is configured for providing power for monopolar electro-cauterization to at least one tool of the tool set, particularly by means of the power supply 220, and for not providing power for monopolar electro-cauterization to still at least one other tool of the tool set, and
   wherein particularly, the system is configured for being operated in at least two surgery modes, the surgery modes comprising a surgery mode for monopolar electro-cauterization.
S65. The system according to any of the two preceding embodiments, wherein the system is configured for providing power for monopolar electro-cauterization to at least one tool of the tool set, power for bipolar electro-cauterization to at least one other tool of the tool set, particularly by means of the power supply 220, and not providing power for electro-cauterization to still at least one other tool of the tool set, and
   wherein the surgery modes of the power supply comprise
   - a surgery mode for monopolar electro-cauterization,
   - a surgery mode for bipolar electro-cauterization, and
   - a surgery mode without cauterization.
S66. The system according to the preceding embodiment and with the features of S56, wherein the device data comprise an indication of a current surgery mode to be activated, such as a surgery mode corresponding to the activated tool.
S67. The system according to any of the preceding system embodiments with the features of S63, wherein the system, particularly the data-processing system 200, is configured for generating electro-cauterization instruction data; wherein the system is configured for providing power for electro-cauterization to the at least one first tool 240 based on the electro-cauterization instruction data by means of the power supply.
S68. The system according to any of the preceding system embodiments with the features of S67 and at least one of S64 and S65, wherein the system, particularly the data-processing system 200, is configured for generating the electro-cauterization instruction data; wherein the system is configured for activating a surgery mode based on the electro-cauterization instruction data.
S69. The system according to any of the two preceding embodiments and with the features of S21 and S34, particularly with the features of S55, wherein the system, particularly the data-processing system 200, is configured for generating the electro-cauterization instruction data at least based on the toolset information data and the alignment data.
S70. The system according to any of the preceding system embodiments with the features of S39 and S67, particularly with the features of S42, wherein the system, particularly the data-processing system 200, is configured for generating the electro-cauterization instruction data at least based on the receiving-location-data.
S71. The system according to any of the preceding system embodiments with the features of S9, S34, S37 and S67, wherein the system, particularly the data-processing system, is configured for generating the electro-cauterization instruction data at least based on the alignment data, and
   wherein the magazine comprises
   - a first set of receiving locations of the magazine only comprising one or more tools for monopolar electro-cauterization, and
   - a second set of receiving locations of the magazine only comprising one or more tools for bipolar electro-cauterization and/or for no electro-cauterization;
   wherein each set of receiving locations of the magazine is arranged at pre-determined regions of the magazine.
S72. The system according to the preceding embodiment, wherein the first set of receiving locations of the magazine only comprises one or more tools for monopolar electro-cauterization, and the second set of receiving locations of the magazine only comprises one or more tools for bipolar electro-cauterization, and a third set of receiving locations only comprises one or more tools not intended for electro-cauterization.
S73. The system according to any of the preceding embodiments and with the features of S51 and S67, wherein the system, particularly the data-processing system 200, is configured for generating the electro-cauterization instruction data at least based on the tool type data generated based on the tool data element of the activated tool.
S74. The system according to any of the preceding system embodiments with the features of S47, S68 and S69, wherein the system is configured for activating the surgery mode for monopolar electro-cauterization based on the toolset information data and the alignment data, particularly based on the tool type data generated based on the toolset information data and the alignment data, and further only if the receiving-location-data corresponding to an activated tool indicate that the mounted tool is configured for monopolar electro-cauterization.
S75. The system according to the preceding system embodiment, wherein the system is configured for activating the surgery modes for bipolar electro-cauterization and no electro-cauterization based on the toolset information data and the alignment data, particularly based on the tool type data generated based on the toolset information data and the alignment data.
S76. The system according to any of the two preceding embodiments and with the features of S46, wherein the step of only activating the surgery mode for monopolar electro-cauterization if the receiving-location-data corresponding to an activated tool indicate that the mounted tool is configured for monopolar electro-cauterization comprises the switching component inhibiting activation of the surgery mode for monopolar electro-cauterization and/or inhibiting power transmission from the power supply to the at least one tool.
S77. The system according to any of the preceding system embodiments with the features of S47, S69 and S71, wherein the system is configured for only activating the surgery mode for monopolar electro-cauterization if a tool from the first set of receiving locations is activated, and
   wherein the system, particularly the data-processing system, is configured for activating the surgery modes for bipolar electro-cauterization and no electro-cauterization based on the toolset information data and the alignment data, particularly based on the tool type data generated based on the toolset information data and the alignment data.
S78. The system according to any of the preceding system embodiments with the features S64, wherein the system, particularly the power supply 220, is configured for determining, upon a begin of bipolar electro-cauterization, whether the tool 240 mounted to the distal portion of the patient component 110 is currently suitable for bipolar electro-cauterization based on at least one electrical property of said tool, such as a resistance and/or an impedance of the tool mounted to the distal portion of the patient component.
S79. The system according to any of the preceding system embodiments with the features of S9, wherein the system is configured for preventing a movement of the receiving locations, particularly of the magazine 242, with respect to the tool activation mechanism 120 while a tool 240 is mounted to the distal portion of the patient component 110.
S80. The system according to any of the preceding system embodiments with the features of S52 and S63, particularly with the features of S26, wherein the system, particularly the data-processing system, is configured for storing settings for electro-cauterization for at least one of the tools for monopolar and/or at least one of the tools for bipolar electro-cauterization.
S81. The system according to the preceding embodiment, wherein at least one of the settings relates to a single tool.
S82. The system according to any of the two preceding embodiments, wherein at least one of the settings relates to all tools for monopolar/bipolar electro-cauterization.
S83. The system according to any of the preceding system embodiments with the features of S63, particularly with the features of S80, wherein the system, particularly the data-processing system 200, is configured for receiving the settings for electro-cauterization by a user input, such as a voice input and/or a button interface of the surgery device.
S84. The system according to any of the preceding system embodiments with the features of S80, wherein the system is configured for providing power to the tools by means of the power supply based on the stored settings.
S85. The system according to any of the preceding system embodiments, wherein the surgery device is a microinvasive surgery device.
S86. The system according to any of the preceding system embodiments with the features of S7, wherein the system, particularly the data-processing system 200, is configured for receiving at least one tool selection input from a user, the tool selection input indicative of a tool to be mounted.
S87. The system according to the preceding embodiment, wherein the system, particularly the data-processing system 200, is configured for receiving the at least one tool selection input by means of a voice input and/or a button interface of the surgery device.
S88. The system according to any of the preceding system embodiments with the features of S86, wherein the system is configured for mounting a selected tool in response to receiving a tool selection input from the user.
S89. The system according to any of the preceding system embodiments, wherein the system, particularly the data-processing system 200, is configured for generating a tool suggestion and outputting the tool suggestions.
S90. The system according to the preceding embodiment, wherein generating the tool suggestion is based on tool use data relating to a use of tools and/or the surgery device.
S91. The system according to any of the preceding system embodiments, wherein the system is configured for carrying out the method according to any of the method embodiments.
M77. The method according to any of the preceding method embodiments, wherein the method comprises using a system according to any of the system embodiments.

Exemplary features of the invention are further detailed in the figures and the below description of the figures.

### Brief description of the figures

Fig. 1 shows a view of a surgery device
Fig. 2 shows an exploded view of the surgery device
Fig. 3 shows a system comprising the surgery device
Figs. 4a, 4b show parts of the tool activation system
Figs. 5a-5d show different cross-sectional views of the surgery device
Figs. 6a-6f show different views of the magazine
Figs. 7a-7b show a magazine, tools and a section of a rack in different configurations
Figs. 8a-8b show the magazine, the tools and the section of the rack in a first configuration
Figs. 9a-9b show the magazine, the tools and the section of the rack in a second configuration
Figs. 10a-10bshow the magazine, the tools and the section of the rack in a third configuration
Fig. 11 shows a section of a patient component and two tools
Figs. 12a-12c show views of a distal portion of the tube component
Figs. 13a-13bshow an embodiment of a tool for bipolar cauterization and a corresponding insulation of tool parts
Figs. 14a-14bshow embodiments of the magazine and an identification element
Figs 15-16 show different embodiments of the system

### Detailed figure description

For the sake of clarity, some features may only be shown in some figures, and others may be omitted. However, also the omitted features may be present, and the shown and discussed features do not need to be present in all embodiments.

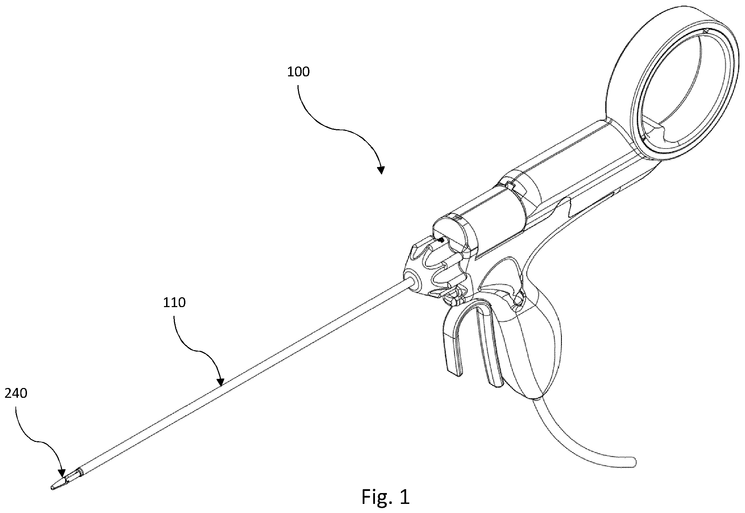

Fig. 1 shows a surgery device 100. In the example of Fig. 1, the surgery device 100 is a microinvasive surgery device. The surgery device 100 of Fig. 1 is configured for performing microinvasive surgical procedures, such as a laparoscopy. The microinvasive surgery device 100 of Fig. 1 is configured for introducing a tool 240 into a body, e.g., of a human, or in another example, into a body of an animal. The tool 240 is mounted at a distal portion of a patient component 110. In the example of Fig. 1, the patient component 110 is a tube component. In particular, the distal portion of said tube component is configured for also being introduced in the body of the human or the animal, allowing to reach a point of surgery remote from an incision through which the tool 240 is introduced.

In the example of Fig. 1, the surgery device 100 is a handheld microinvasive surgery device 1. In the prior art, handheld microinvasive surgery devices comprise one tool 40. If a different tool 240 is to be used, the previously used surgery devices are removed from the body of the human or the animal, and a different handheld device is introduced through the incision into the body, resulting in a prolonged time in operation, in which, e.g., the patient needs to be under anaesthetic. Also, more staff is necessary, e.g., for providing different surgery devices 100 to a surgeon performing a surgery.

The surgery device 100 in Fig. 1 comprises a plurality of tools 240. The surgery device 100 is configured for changing a mounted tool 240 while the distal portion of the patient component 110 is located inside of the body of the patient.

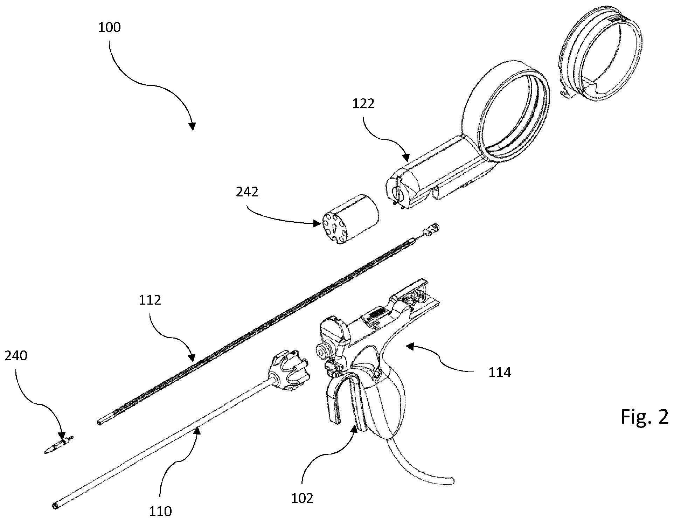

Fig. 2 shows an exploded view of the surgery device 100. As can be seen, the surgery device 1 shown in Fig. 2 comprises the patient component 110, the tool 240, a rack 112, a magazine 242, a body element 114 and a motor system 122. Further, the microinvasive surgery device 1 further comprises a rack receptacle at its proximal end. The rack receptacle is shown in an opened configuration. Further, an operating lever 102 of the surgery device 1 can be seen in Fig. 2. As in Fig. 1, in the example of Fig. 2, the surgery device 100 is a microinvasive surgery device.

As can be seen, the patient component 110 is configured to be mounted to the body element 114. The orientating formation thus allows to turn the patient component 110 about a longitudinal axis of the patient component.

In the example of Fig. 2, the body element 114 comprises a handle as well as the operating lever 102 and a connection cable for connecting the surgery device 100 to a power supply 220, a screen 210 and a data-processing system 200 (not shown in Fig. 2).

The surgery device 100 is configured for changing a mounted tool, i.e., a tool 240 at the distal portion of the patient component 110. The distal portion of the patient component 110 is the portion facing the patient's body in a use configuration of the surgery device 100.

In the example of Fig. 2, the motor system 122 comprises a magazine drive configured to rotate the magazine 242 and a rack drive configured to enable changing the mounted tool 240. The motor system 122 is configured for moving the magazine 242 to a rotatory position, that is, an angular position. Thus, optionally advantageously, the motor system 122 may be configured for aligning a chamber of the magazine 242 with the patient component 110.

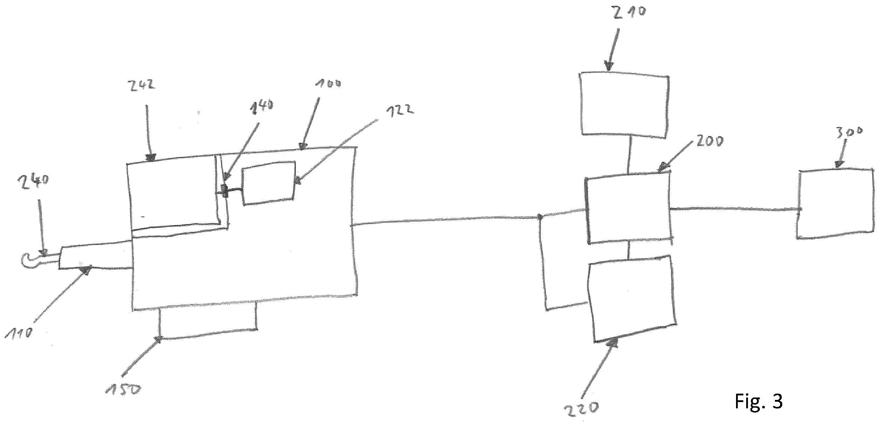

Fig. 3 is a schematic representation of a system comprising the surgery device 100. In Fig. 3, the surgery device 100 comprises a patient component 110 configured for being introduced in the body of the human or the animal. Further, in a configuration shown in Fig. 3, a tool 240 is mounted to the distal portion of the patient component 110. Further, as can be seen, the surgery device 100 comprises a toolset interface 140 configured for receiving a tool set comprising a plurality of tools 240.

In the example of Fig. 3, a magazine 242 comprises the tool set, and the tool set interface 140 is configured for receiving the magazine 242 comprising the tool set. Further, the surgery device 100 of Fig. 3 comprises a button interface 150 configured to receive inputs from a user of the surgery device 100. By means of the button interface 150, for example, a change of tools 240 of the surgery device 100 and/or other functions of the surgery device 100, such as an electro-cauterization function are controlled.

Further, in the example of Fig. 3, the system comprises a data-processing system 200, a screen 210, a power supply 220 and a connection to a server 300. The data-processing system 200 further comprises a data-exchange component 212 configured to at least receive data from an identification element 252.

The data-processing system 200 may comprise one or more processing units configured to carry out computer instructions of a program (i.e. machine readable and executable instructions). The processing unit(s) may be singular or plural. For example, the data-processing system 200 may comprise at least one of CPU, GPU, DSP, APU, ASIC, ASIP or FPGA. The data-processing system 200 may comprise memory components, such as, main memory (e.g. RAM), cache memory (e.g. SRAM) and/or secondary memory (e.g. HDD, SDD). The data-processing system 200 may comprise volatile and/or non-volatile memory such an SDRAM, DRAM, SRAM, Flash Memory, MRAM, F-RAM, or P-RAM. The data-processing system 200 may comprise internal communication interfaces (e.g. busses) configured to facilitate electronic data exchange between components of the data-processing system 200, such as, the communication between the memory components and the processing components. The data-processing system 200 may comprise external communication interfaces configured to facilitate electronic data exchange between the data-processing system 200 and devices or networks external to the data-processing system 200, such as the identification element 252 or the server 300. For example, the data-processing system 200 may comprise network interface card(s) that may be configured to connect the data-processing system 200 to a network, such as, to the Internet. The data-processing system 200 be configured to transfer electronic data using a standardized communication protocol. The data-processing system 200 may be a centralized or distributed computing system.

In Fig. 3, the data-processing system 200 is shown separate from the surgery device 100. For example, the data-processing system 200 may be packaged in a console comprising a connection to the surgery device 100, the server 300 and the power supply 220.

However, in another embodiment not shown, the surgery device 100 comprises a part of the data-processing system 200. For example, the surgery device 100 may comprise one or more controllers of the data-processing system 200 or the whole data-processing system 200. However, a part of the data-processing system may also be packaged with the power supply 220 or still in another part of the system.

Further, returning to the data-processing system 200, the data-processing system may comprise user interfaces, such as:
- output user interface, such as:
   ∘ screens or monitors configured to display visual data (e.g. displaying graphical user interfaces of the questionnaire to the user),
   ∘ speakers configured to communicate audio data (e.g. playing audio data to the user),
- input user interface, such as:
   ∘ a camera configured to capture visual data (e.g. capturing images and/or videos of the user or an inside of the body of the human or the animal),
   ∘ a microphone configured to capture audio data (e.g. recording audio instructions from the user),
   ∘ the button interface 150,
   ∘ a keyboard configured to allow the insertion of text and/or other keyboard commands (e.g. allowing the user to enter text data and/or other keyboard commands by having the user type on the keyboard) and/or
   ∘ a trackpad, mouse, touchscreen, joystick - configured to facilitate the navigation through different graphical user interfaces of the questionnaire.

To put it simply, the data-processing system 200 may be a processing unit configured to carry out instructions of a program. The data-processing system 200 may be a system-on-chip comprising processing units, memory components and busses. The data processing system may be a personal computer, a laptop, a pocket computer, a smartphone, a tablet computer. The data-processing system 200 may be a processing unit or a system-on-chip that may be interfaced with a personal computer, a laptop, a pocket computer, a smartphone, a tablet computer and/or user interfaces (such as the upper-mentioned user interfaces).

With continued reference to the example of Fig. 3, the power supply 220 is a high-frequency generator for monopolar and bipolar electro-cauterization, also referred to as electrosurgical generator. In the system shown in Fig. 3, the power supply 220 is configured for providing power for monopolar electro-cauterization and power for bipolar electro-cauterization to tools 240 of the tool set.

An example of such a power supply 220 is be a high-frequency electro-surgical generator, such as ERBE vio 300, commercially available from Erbe Elektromedizin GmbH, Tübingen, Germany.

The power supply 220 may for example satisfy the requirements of EN IEC 60601-2-2:2018-12 regarding overload and protection against the effects of short-circuiting of the electrodes.

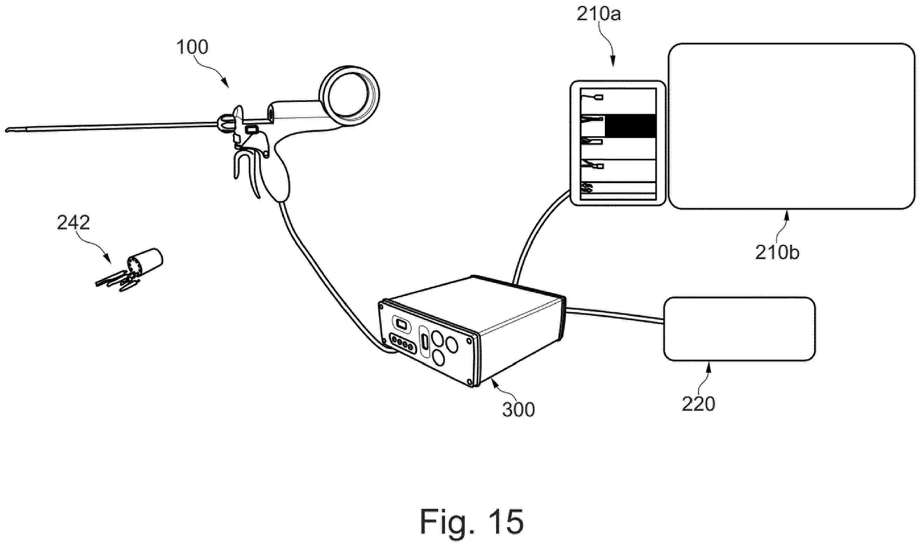

An embodiment of the system is shown in Fig. 15. In Fig. 15, the system comprises the microinvasive surgery device 100, the magazine 242 comprising the tool set 241, the power supply 220, the data-processing system 200 and two screens 210a, 210b.

In the example of Fig. 15, the data-processing system 200 is packaged in a console. The console comprises connections to the microinvasive surgery device 100, a first screen 210a and the power supply 220. Further, the console may comprise connections to the power grid and/or the server 300 (not shown in Fig. 15).

In the example of Fig. 15, the first screen 210a outputs data relating to the tool 240 mounted to the distal portion of the patient component 110, as well as to other tools 240 of the tool set 241, e.g., a list of tools 240 in the tool set 241 or the magazine 242. However, other data relating to the system, e.g., a setting of the power supply 220 or an electro-cauterization setting may also be output. In the example of Fig. 15, the output of the first screen 210a is controlled by means of the data-processing system 200.

Still relating to the embodiment shown in Fig. 15, the second screen 210b is configured for outputting image data captured by a camera of the system (not shown). The camera may for example be a camera for microinvasive surgery configured to image a region inside of a body of the patient.

By using two screens, one for the image data from the camera and one for the data relating to the tools, a stable operation of the system may be improved. Further, thus, the system can be used to retrofit/upgrade an existing surgery equipment set.

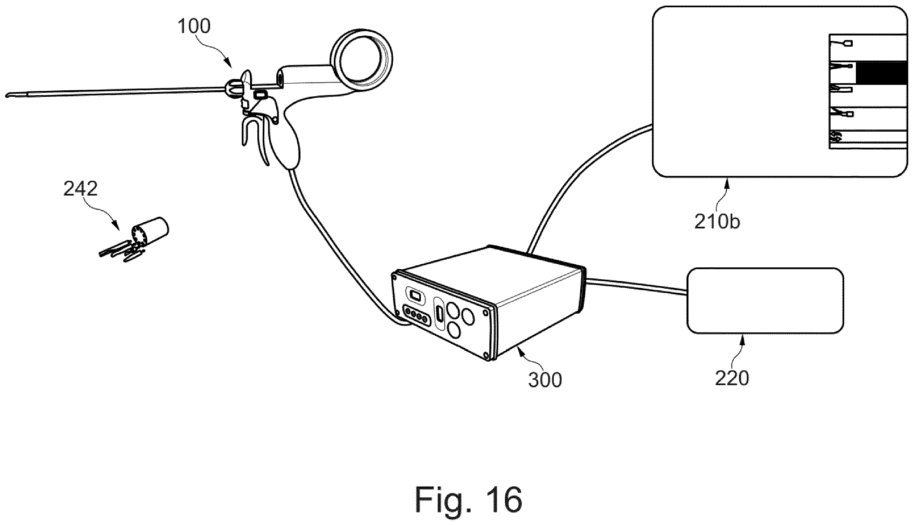

Another embodiment of the system is shown in Fig. 16. In Fig. 16, the system comprises the microinvasive surgery device 100, the magazine 242 comprising the tool set 241, the power supply 220, the data-processing system 200 and a screen 210b.

As in Fig. 15, in Fig. 16, the data-processing system 200 is packaged in a console. The console comprises connections to the microinvasive surgery device 100, the screen 210b and the power supply 220. Further, as in Fig. 16, the console may comprise connections to the power grid and/or the server 300 (not shown in Fig. 16).

The screen 210b in Fig. 16 is configured for outputting image data captured by the camera of the system, such as the camera for microinvasive surgery configured to image a region inside of a body of the patient.

Further, in the embodiment shown in Fig. 16, the screen 210b is configured for outputting the data relating to the tool 240 mounted to the distal portion of the patient component 110, as well as to other tools 240 of the tool set 241, e.g., the list of tools 240 in the tool set 241 or the magazine 242 together with the image data captured by the camera. These data may for example be outputted as an overlay to the image data captured by the camera.

By using one screen and visualizing the data relating to the tools 240 together with the camera data, an integrated, space-efficient and easy-to-use solution may optionally advantageously be provided. Further, less devices are necessary, which may be optionally advantageous in a surgery room.

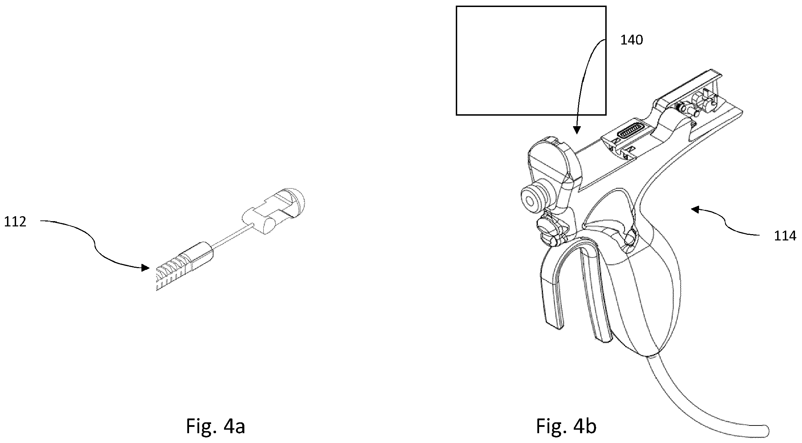

Figs. 4a and 4b show a detail of the rack 112 and the body element 114. In the example of Fig. 4b, a part of the toolset interface 140 is shown. In this example, the toolset interface 140 is configured for receiving the magazine 242 comprising the tool set 241, which tool set 241 comprises a plurality of tools 240.

Further, in Fig 4b, the motor system 122 is dismounted from the surgery device 100. However, the motor system may also form part of the toolset interface 140.

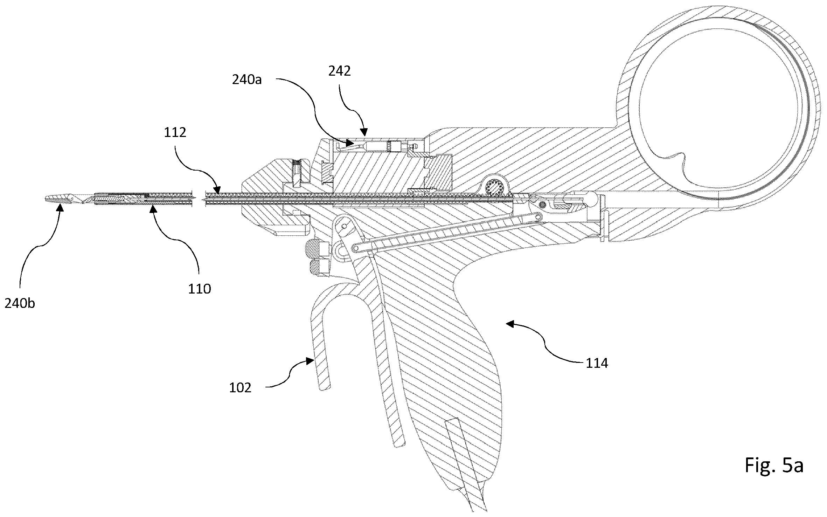

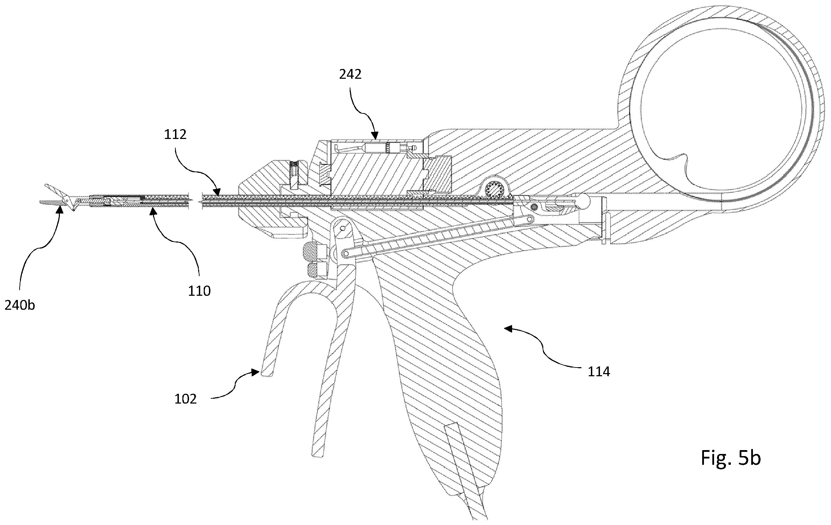

Figs. 5a-5d show different cross-sectional views of the surgery device 100. In Figs. 5a and 5b, a forceps tool 240b is mounted to the distal portion of the patient component 110 by means of the rack 112. While a rack 112 is shown, different mounting mechanisms can as well be conceived, e.g. as disclosed in EP24151941.2.

Further, the magazine 242 is mounted to the surgery device 100, more particularly to the toolset interface 140. While the above-mentioned tool 240b from the magazine 242 is mounted to the distal portion of the patient component 110, a hook tool 240a is located in the magazine 242 in the configuration shown in Fig. 5a.

As can be seen from Figs. 5a and 5b, by means of the operating lever 102, the tool 240b can be mechanically actuated. However, the person skilled in the art will easily understand that some tools are not suitable for being mechanically actuated, as they do not comprise separate parts, such as the hook tool 240a shown.

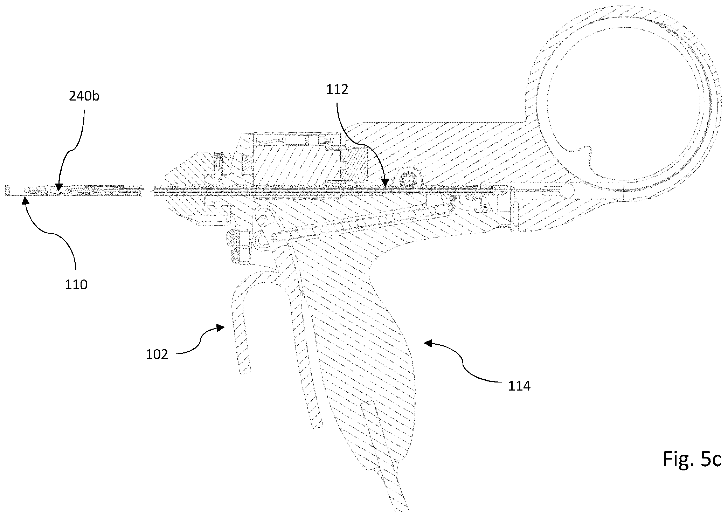

Fig. 5c shows the surgery device 100 in a configuration where the tool 240b is dismounted from the distal portion of the patient component 110 and retracted towards the magazine 242.

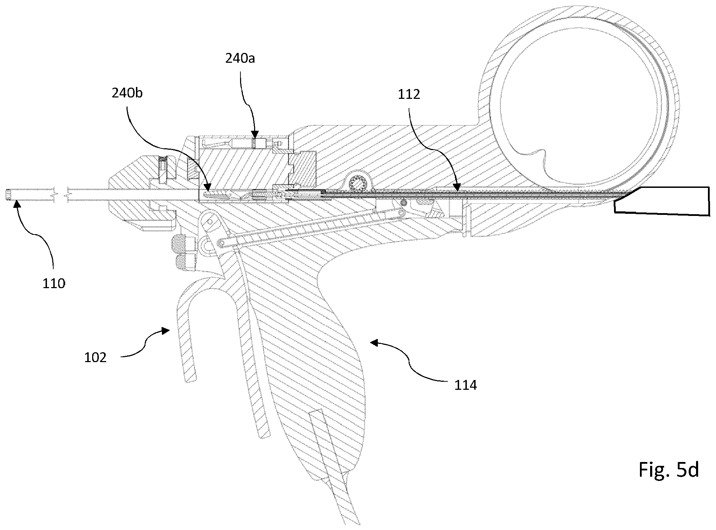

In Fig. 5d, a configuration is shown in which the tool 240a is fully retracted into the magazine 242 and the tool 240 to be mounted can be changed. To change the tool 240, the motor system 122 rotates the magazine so that another chamber of the magazine 242 is aligned with the patient component 110, such as the chamber with the hook tool 240a. Once the chamber with the other tool 240b is aligned with the patient component 110, said other tool, such as the hook tool, can be mounted to the distal portion of the patient component 110.

In Figs. 5a-5d, at least one of the plurality of tools 240 is a multi-part-tool. The multi-part-tool comprises at least one movable tool part, as can be seen, e.g., in Fig. 5b. The at least one moveable tool part is configured for being moved relative to another part of the tool, such as a remainder of the tool or a second moveable part of the tool.

In the example of Fig. 5b, the movable part of the tool 240b is a jaw of the forceps tool. The remainder is a corresponding jaw of the tool 240b. Also, in the example of Fig. 5b, a second moveable part in a kinematic chain with the first part can be seen.

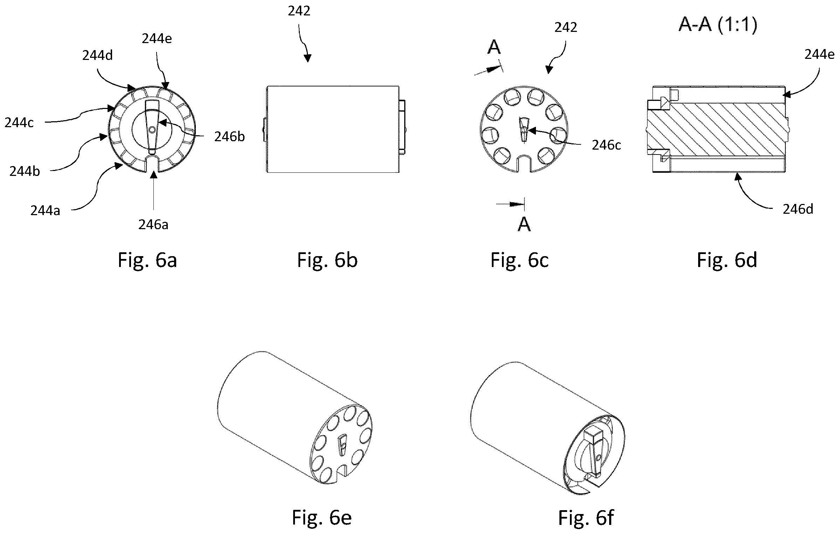

Figs. 6a-6f show different views of an exemplary magazine 242 comprising a plurality of receiving locations 244a, 244b, 244c, 244d, 244e.

As can be seen, the exemplary magazine 242 comprises a plurality of receiving locations 244a-244e, which, in the example of Figs. 6a-6f, are chambers of the magazine 242. Each receiving location 244a-244e is configured for receiving a tool 240 from the tool set 241.

Further, in the example shown in Figs. 6a-6f, the magazine 242 comprises a plurality of magazine coupling elements 246a-246d. The magazine coupling elements 246a-246d allow to couple the magazine 242 to the toolset interface 140, and optionally to the motor system 122.

In an example, at least one of the magazine coupling elements 246b, 246c comprises a wedge shape. Thus, optionally advantageously, an angular position of the magazine 242 with respect to a corresponding coupling element of the motor system 122 may be constant and an angular or rotatory position of the magazine 242 may be estimated based on a angular or rotatory position of the motor system 122.

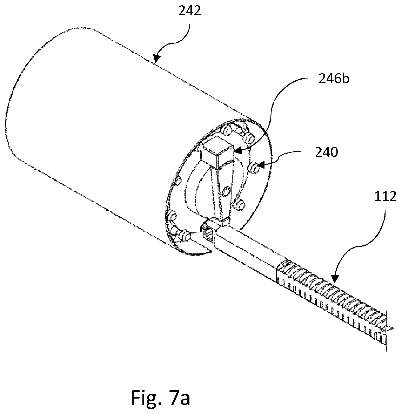

Figs. 7a-9b show how the magazine 242 is rotated so as to align a selected tool 240b with the patient component 110 (not shown in Figs. 7a-10b for intelligibility).

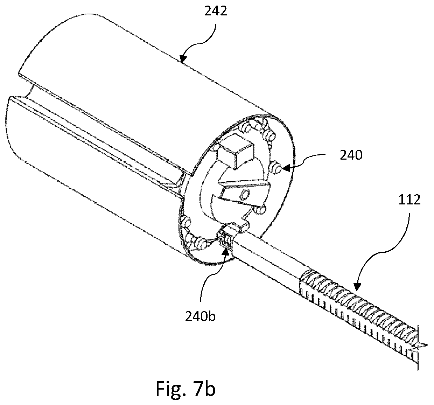

Fig. 7a shows the magazine 242 in a neutral configuration, in which no tool is aligned with the patient component. In Fig. 7b, a receiving location with a selected tool 240b, in the example of Figs. 7a-9b a forceps tool, is aligned with the patient component 110.

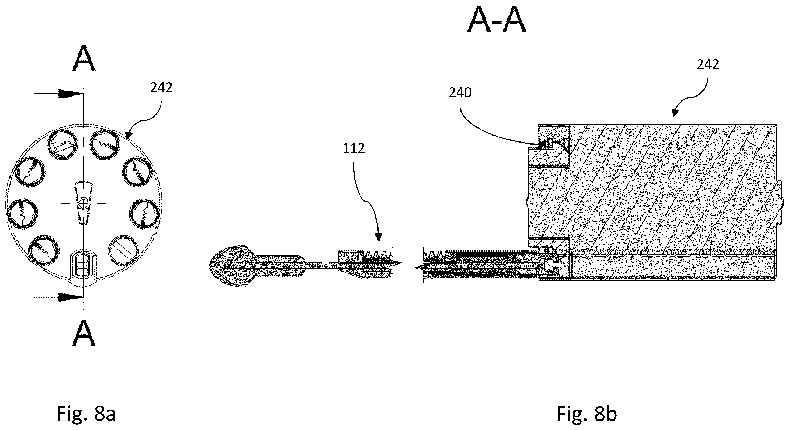

Figs. 8a and 8b show different views of the neutral configuration.

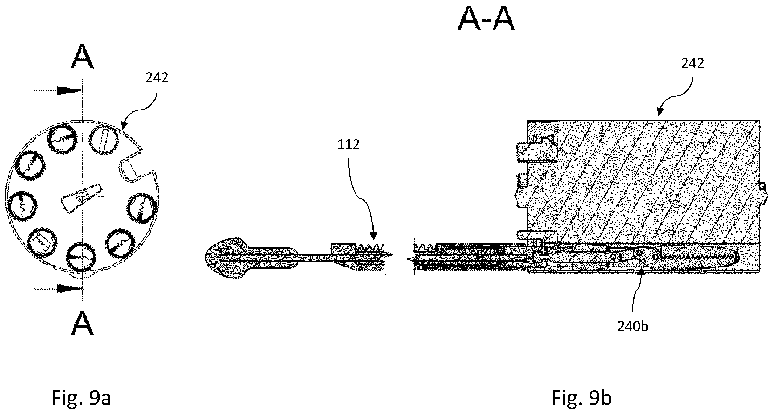

Figs. 9a and 9b show different views of the configuration in which the selected tool 240b is aligned with the patient component 110.

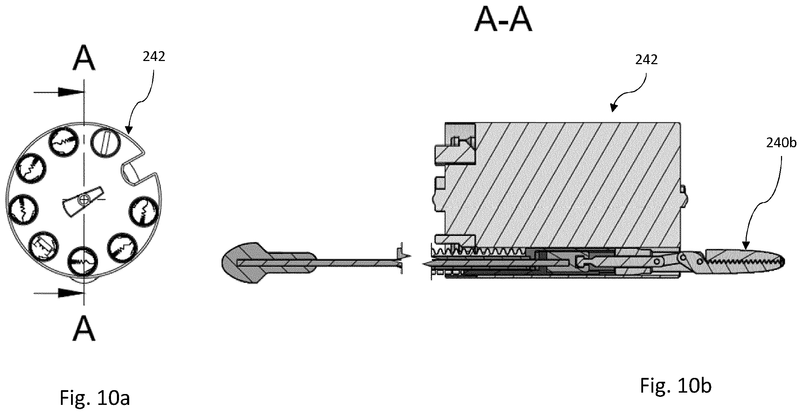

Figs. 10a and 10b show views of the selected tool 240b being mounted to the distal portion of the patient component 110. In the example of Fig. 10b, the rack 112 moves the tool 240b from the magazine 242 to the distal portion of the patient component 110. Analogously, the selected tool 240b can be dismounted again from the distal portion of the patient component 110 and, for example, be retracted to the magazine 242 for further use, for sterilization or for discarding or further processing after an end of a surgical procedure.

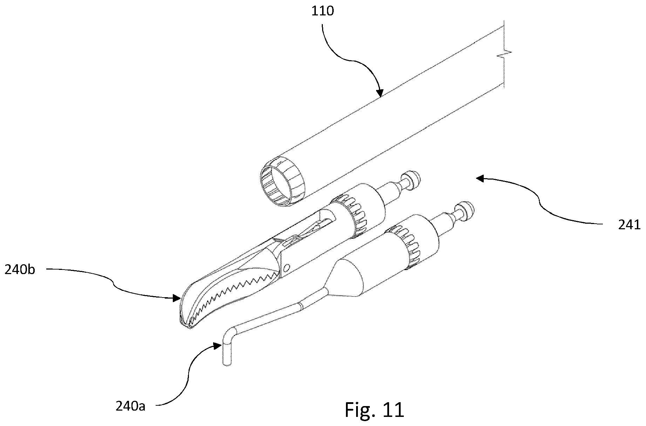

Fig. 11 shows the distal portion of the patient component 110 as well as a tool set 241 comprising two tools 240a, 240b. In the example of Fig. 11, the tool set 241 comprises a hook tool and a forceps tool. However, the tool set 241 may also comprise more or different tools 240.

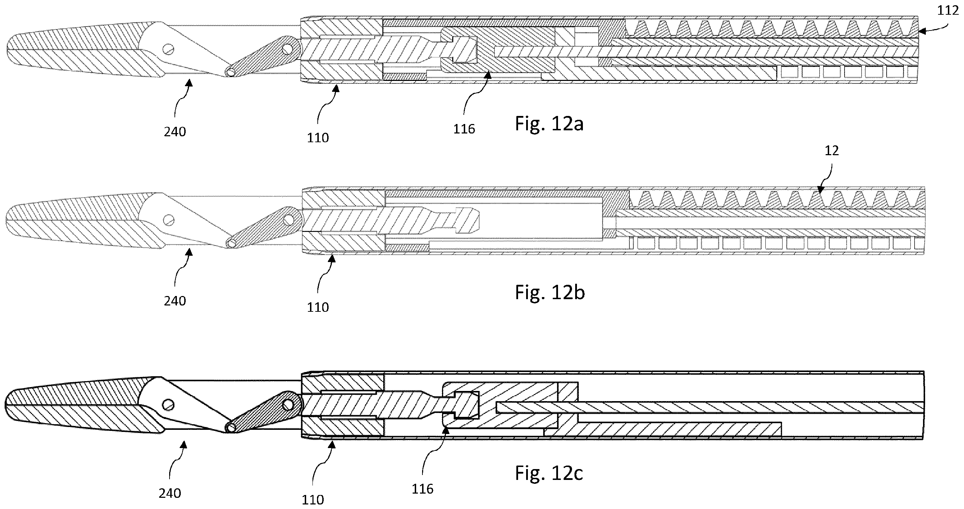

Fig. 12a is a cross-sectional view of the distal portion of the patient component 110 with a tool 240 mounted. In this cross-sectional view of the distal portion of the patient component 110, the tool 240, a tool coupling element 116, the rack 112 and a rod can be seen.

Fig. 12b is another cross-sectional view of the distal portion of the patient component 110 with the tool 240 mounted. In contrast to Figs. 12a and 12c, for clarity and easier understanding of the invention, the tool coupling element 116 and the rod are not shown, but only the tool 240, the patient component 110 and the rack 112.

Fig. 12c shows another cross-sectional view of the distal portion of the patient component 110 with the tool 240 mounted. For clarity and easier understanding of the invention, the rack 112 is not shown, but only the patient component 110, the tool 240, the tool coupling element 16 and the rod.

The person skilled in the art easily understands that, in the example shown in Figs. 12a-12c, the patient component 110 is a tube component.

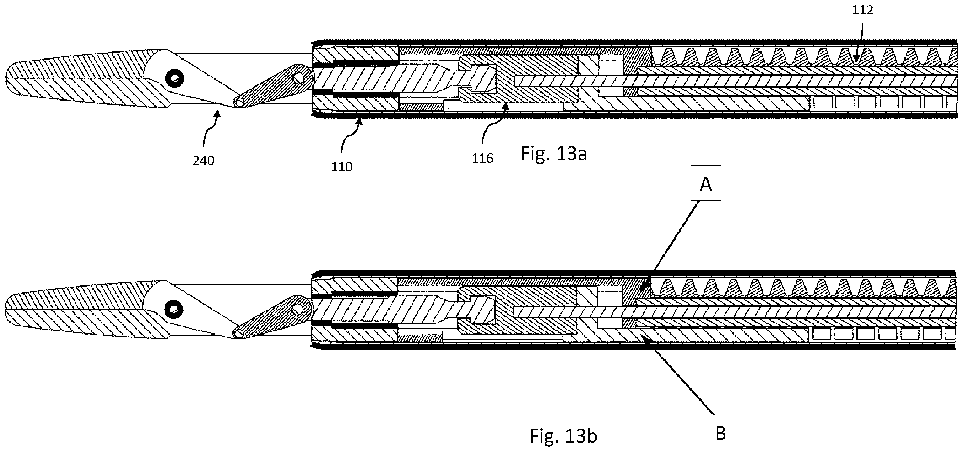

Figs. 13a-13b show an example embodiment of the surgery device 100 configured for bipolar electric cauterization. In the example of Figs. 13a-13b, a multi-part-tool 240, e.g., a forceps, configured for bipolar cauterization is mounted to the distal end of the patient component 110. In this example, two pole lines transmit power to different parts of the forceps, e.g., a first and a second jaw.

In the example shown, the patient component 110 is the tube component. The tube component is configured for transmitting electric power to the tool 240 and thus optionally forms a first pole-line. Further referring to the example shown, the rod also transmits electric power to the tool 240 and thus forms a second pole-line.

Further, in Figs. 13a and 13b, electrically insulating layers are indicated by bold lines. The tube component comprises an outer electrically insulating layer. Further, in case of a tool 240 for bipolar electric cauterization, parts of the tool may be insulated from each other, as the jaws of the forceps as well as the parts connected to the pole-lines. In case of a tool for monopolar cauterization, such insulation may not be necessary.

In the example embodiment shown in Figs. 13a-13b, the body element 114 may comprise a bore configured for receiving the patient component 110, which may form a sleeve bearing. The bore may be made from a conductive material, such as stainless steel. The bore may be configured for transmitting electric power from the body element 114 to the patient component 110, such as the tube component.

A distal end portion A of the rack 112 and a sliding member B of the tool coupling element 116 may be made from an electrically insulating material, as shown in Fig. 13b.

Hence, optionally advantageously, the surgery device 100 may be configured for mounting and operating tools 240 configured for electric cauterization additionally or alternatively to tools that are not configured for electric cauterization when connected to an appropriate power generator.

Further, the person skilled in the art will easily understand that the above-discussed example also allows to provide power to a tool configured for monopolar electric cauterization.

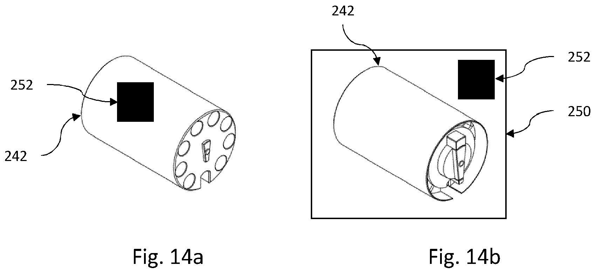

Figs. 14a and 14b show different embodiments of the magazine 242 and an identification element 252.

In the example of Figs. 14a-14b, each tool set 241, more particularly, each magazine containing a tool set 241, is associated with an identification element.

The identification element is configured to store data relating to the tool set 241. The data-exchange component 212 of the data-processing system 200 is configured for reading said data relating to the tool set 241. The identification element 252 can be a known element for data storage, such as optically encoded data, e.g. a barcode, a QR-code, an RFID-chip or the like.

In the embodiment shown in Fig. 14a, the identification element 252 is located on or in the magazine 242, e.g., glued onto the magazine 242 or marked to a surface of the magazine, such as by laser marking.

In the example of Fig. 14b, a packaging 250 of the magazine 242 comprises the identification element 252. The packaging 250 is a sterile packaging, allowing for keeping the magazine 242 inside sterile. Thus, optionally advantageously, the identification element 252 may be read in an environment that does not need to be sterile without comprising sterility of the magazine 242 and/or the tool set 241.

While in the above, a preferred embodiment has been described with reference to the accompanying drawings, the skilled person will understand that this embodiment was provided for illustrative purpose only and should by no means be construed to limit the scope of the present invention, which is defined by the claims.

Whenever a relative term, such as "about", "substantially" or "approximately" is used in this specification, such a term should also be construed to also include the exact term. That is, e.g., "substantially straight" should be construed to also include "(exactly) straight".

Whenever steps were recited in the above or also in the appended claims, it should be noted that the order in which the steps are recited in this text may be accidental. That is, unless otherwise specified or unless clear to the skilled person, the order in which steps are recited may be accidental. That is, when the present document states, e.g., that a method comprises steps (A) and (B), this does not necessarily mean that step (A) precedes step (B), but it is also possible that step (A) is performed (at least partly) simultaneously with step (B) or that step (B) precedes step (A). Furthermore, when a step (X) is said to precede another step (Z), this does not imply that there is no step between steps (X) and (Z). That is, step (X) preceding step (Z) encompasses the situation that step (X) is performed directly before step (Z), but also the situation that (X) is performed before one or more steps (Y1), ..., followed by step (Z). Corresponding considerations apply when terms like "after" or "before" are used.

### Numbered reference signs

- 100: surgery device
- 102: operating lever
- 110: patient component/tube component
- 112: rack
- 114: body element
- 116: tool coupling element
- 120: tool activation system
- 122: motor system
- 140: toolset interface
- 150: button interface
- 200: data-processing system
- 210: screen
- 212: data-exchange component
- 220: power supply
- 240: tool
- 241: tool set
- 242: magazine
- 244: receiving locations
- 246: magazine coupling
- 250: packaging
- 252: identification element
- 300: server

## Claims

1. A system, comprising
- a surgery device (100);
- a tool set (241) comprising a plurality of tools (240);
- a power supply (220) configured for providing electric power for electric cauterization;
- a magazine (242) comprising the tool set (241); and
- a data-processing system (200);
wherein the surgery device comprises
- a body element (114);
- a patient component (110) comprising a distal portion;
- a toolset interface (140) configured for receiving a magazine (242) comprising the tool set (241);
- a tool activation system (120), wherein the tool activation system (120) is configured for mounting at least one tool (240) of the tool set to the distal portion of the patient component (110); and
- an operating element;
wherein the distal portion of the patient component is configured to be introduced into a body of a patient; and
wherein the magazine is configured to be mounted to the toolset interface of the surgery device.

2. The system according to the preceding claim, wherein the magazine (242) comprises a plurality of receiving locations (244), such as a plurality of chambers;
wherein the surgery device comprises a motor system (122), wherein the motor system (122) is configured for moving the magazine (242) coupled to the body element (114) with respect to the body element (114),
wherein activating the at least one tool (240) comprises aligning one of the receiving locations (244) with the tool activation mechanism (120), particularly with the patient component (110); and
wherein the motor system (122) is configured for aligning the one of the receiving locations (244) with the tool activation mechanism (120), particularly with the patient component (110).

3. The system according to any of the preceding claims, wherein the data-processing system 200 comprises a data-exchange component 212.

4. The system according to any of the preceding claims, wherein the system, particularly the data-processing system (200), is configured for processing tool set information data relating to the tool set (241) that the magazine (242) comprises;
wherein the system, particularly the data-processing system (200), is configured for processing a tool set identifier;
wherein the system, particularly the data-processing system (200), is configured for determining the toolset identifier based on an identification element (252) associated with the tool set (241); and
wherein particularly, the data-exchange component 212 is configured for reading the toolset identifier from the identification element 252.

5. The system according to any of the preceding claims, wherein the system is configured for generating alignment data,
wherein the alignment data is indicative of the receiving location (244) aligned with the tool activation mechanism, particularly the receiving location (244) aligned with the patient component (110);
wherein particularly, the system is configured for generating the alignment data at least by sensing a position and/or an orientation of the magazine with respect to the tool activation system (120).

6. The system according to any of the preceding claims with the features of claims 2 and 3, wherein the system, particularly the data-processing system (200), is configured for processing receiving-location-data for at least one of the activated tool of the tool set and the receiving location aligned with the tool activation mechanism;
wherein the magazine comprises at least one or a plurality of receiving location elements, wherein each receiving location element is associated with a receiving location;
wherein the system, particularly the data-exchange component (212) and/or a receiving location sensor, is configured for sensing the receiving location element associated with the receiving location aligned with the patient component and/or a presence of a receiving location element associated with the receiving location aligned with the patient component; and
wherein the system is configured for generating the receiving-location-data based on the sensed receiving location element.

7. The system according to any of the preceding claims, wherein the system is configured for providing power for monopolar electro-cauterization to at least one tool of the tool set, particularly by means of the power supply (220), and for not providing power for monopolar electro-cauterization to still at least one other tool of the tool set; and
the system is configured for being operated in at least two surgery modes, the surgery modes comprising a surgery mode for monopolar electro-cauterization.

8. The system according to the preceding claim and with the features of claims 4 and 5, particularly further with the features of claim 6, wherein the system, particularly the data-processing system (200), is configured for generating electro-cauterization instruction data; wherein the system is configured for providing power for electro-cauterization to an at least one first tool (240) based on the electro-cauterization instruction data by means of the power supply;
wherein the system, particularly the data-processing system (200), is configured for generating the electro-cauterization instruction data at least based on (i) the toolset information data and the alignment data and/or (ii) the receiving location data.

9. The system according to the preceding claim and with the features of claim 6, wherein the receiving-location-data indicate at least whether a tool is configured
(a) for monopolar electro-cauterization or
(b) for at least one of bipolar electro-cauterization and/or no electro-cauterization,
wherein the system, particularly the data-processing system 200, is configured for generating the electro-cauterization instruction data; wherein the system is configured for activating a surgery mode based on the electro-cauterization instruction data;
wherein the system is configured for activating the surgery mode for monopolar electro-cauterization based on the toolset information data and the alignment data, particularly based on the tool type data generated based on the toolset information data and the alignment data, and further only if the receiving location data corresponding to an activated tool indicate that the mounted tool is configured for monopolar electro-cauterization.

10. The system according to the preceding claim, wherein the system, particularly the surgery device, comprises a switching component configured for inhibiting provision of power to the tool based on the sensed receiving location element;
wherein the system being configured for only activating the surgery mode for monopolar electro-cauterization if the receiving-location-data corresponding to an activated tool indicate that the mounted tool is configured for monopolar electro-cauterization comprises the switching component being configured for inhibiting activation of the mode for monopolar electro-cauterization and/or inhibiting power transmission from the power supply to the at least one tool.

11. The system according to any of claims 4-10 and with the features of claim 5, wherein the system, particularly the data-processing system (200), is configured for processing tool type data, wherein the tool type data relate to the at least one tool (240) mounted to the distal portion of the patient component (110);
wherein processing the tool type data comprises determining the tool type data relating to the at least one tool mounted to the distal portion of the patient component based on the toolset information data and the alignment data.

12. The system according to any of the preceding claims, wherein the system further comprises a user-interface device;
wherein the user-interface device comprises a camera screen (210) configured for outputting image data generated by the camera for microinvasive surgery,
wherein particularly, the user-interface device further comprises an additional screen (210), wherein the additional screen is connected to the data-processing system (200).

13. The system according to the preceding two claims, wherein the system is configured for outputting device data; wherein the device data comprise an indication of the tool type data;
wherein particularly, the device data comprise an indication of a current surgery mode to be activated, such as a surgery mode corresponding to the activated tool.

14. The system according to the preceding two claims, wherein
- outputting the device data, particularly the indication of the tool type data, comprises outputting the indication of the tool type data together with the image data generated by the camera by means of the camera screen 210, or
- outputting the indication of the tool type data comprises outputting the indication of the tool type data by means of the additional screen connected to the data-processing system 200.

15. The system according to any of the preceding claims, wherein
- the surgery device is a robotic surgery device, particularly a robotic microinvasive surgery device, or
- the surgery device is a handheld device, particularly a handheld microinvasive surgery device.
